# EUROPEAN PATENT SPECIFICATION

(11) **EP 3 526 763 B1**
(45) Date of publication and mention of the grant of the patent: **16.09.2026**
(21) Application number: 18739394.7
(22) Date of filing: 12.01.2018
(51) Int. Cl.: G06Q 50/22, G06F 21/72, G04G 9/00, G16H 10/00, G16H 20/00, G16H 30/00, G16H 40/00, G16H 50/00

(54) **ELECTRONIC DEVICE AND METHOD FOR PROVIDING GUIDE INFORMATION BASED ON GENETIC INFORMATION**
ELEKTRONISCHE VORRICHTUNG UND VERFAHREN ZUR BEREITSTELLUNG VON FÜHRUNGSINFORMATIONEN AUF BASIS VON GENETISCHEN INFORMATIONEN
DISPOSITIF ÉLECTRONIQUE ET PROCÉDÉ DE FOURNITURE D'INFORMATIONS DE GUIDAGE SUR LA BASE D'INFORMATIONS GÉNÉTIQUES

(30) Priority: 13.01.2017 KR 20170006275
(43) Date of publication of application: 21.08.2019
(73) Proprietor: Samsung Electronics Co., Ltd., Suwon-si, Gyeonggi-do 16677 (KR)
(72) Inventor: KANG, Ki-Man, Suwon-si, Gyeonggi-do 16678 (KR); KIM, Tae-Ho, Cheongju-si, Chungcheongbuk-do 28391 (KR); PARK, Jeong-Min, Hwaseong-si, Gyeonggi-do 18477 (KR); LEE, Seung-Eun, Seoul 06608 (KR)
(74) Representative: HGF
(86) International application number: PCT/KR2018/000627
(87) International publication number: WO 2018/131939

(56) References cited:
- KR-A- 20150 075 589
- KR-A- 20160 111 557
- US-A1- 2011 172 497
- US-A1- 2013 138 447
- US-A1- 2015 051 451
- US-A1- 2015 051 451
- US-A1- 2016 217 266
- US-B1- 8 930 204

## Description

### Technical Field

Various exemplary embodiments of the present disclosure relate to an electronic device and a method for providing guide information based on genetic information.

### Background Art

Nowadays, mobile devices including smartphones are essential to daily life. With a growing number of users paying attention to their health information in recent years, healthcare management of things such as exercise, food, and skin care services related to health can be provided using smartphones. For example, a mobile device can obtain a user's body information (for example, height, weight, heart rate, blood sugar level, or blood pressure level), the user's exercise information (for example, a step count), and the like using various sensors provided in the mobile device and can provide information necessary for the user's healthcare based on the obtained information.

US 2016/217266 A1 ((DAMANI SAMIR B [US] ET AL) 28 July 2016 (2016-07-28) describes a system that dynamically selects and updates personalized health programs based on real-time biometric data from wireless devices. It automatically adjusts wellness interventions for users based on incoming activity metrics.

US 2015/051451 A1 (KIDO TAKASHI [JP]) 19 February 2015 (2015-02-19) details a method for prediction of disease risk.

### Disclosure of Invention

### Technical Problem

Various exemplary embodiments may provide an electronic device for providing not only a user's health information but also guide information for the user's healthcare based on genetic information and status information corresponding to an activity or a state of the user, and a method thereof.

### Solution to Problem

Various exemplary embodiments may provide an electronic device providing guide information based on genetic information, and a method thereof.

An electronic device, a computer-implemented method and a non-transitory computer-readable medium are defined in the appended claims.

### Advantageous Effects of Invention

According to various exemplary embodiments of the present disclosure, more accurate and useful healthcare services may be provided for a user based on genetic information on the user and status information corresponding to the activity or the state of the user as defined in the appended claims.

### Brief Description of Drawings of the disclosure

For a more complete understanding of the present disclosure and its advantages, reference is now made to the following description taken in conjunction with the accompanying drawings, in which like reference numerals represent like parts:
FIG. 1 illustrates a network environment including an electronic device according to various exemplary embodiments of the present disclosure;
FIG. 2 illustrates a block diagram of an electronic device according to various exemplary embodiments of the present disclosure;
FIG. 3 illustrates a block diagram of a program module according to various exemplary embodiments of the present disclosure;
FIGS. 4A and 4B illustrate configurations of a system according to various exemplary embodiments of the present disclosure;
FIG. 5 illustrates the configuration of an electronic device according to various exemplary embodiments of the present disclosure;
FIG. 6 illustrates the configuration of a first server according to various exemplary embodiments of the present disclosure;
FIG. 7 illustrates an example of a table according to various exemplary embodiments of the present disclosure;
FIG. 8 illustrates a flowchart for providing guide information based on genetic information and status information according to various exemplary embodiments of the present disclosure;
FIGS. 9A and 9B illustrate flowcharts for providing guide information based on genetic information and status information according to various exemplary embodiments of the present disclosure;
FIG. 10 illustrates a flowchart for providing guide information based on genetic information and status information according to various exemplary embodiments;
FIGS. 11A, 11B, and 11C illustrate examples of a user interface associated with a healthcare service according to various exemplary embodiments of the present disclosure;
FIGS. 12A and 12B illustrate examples of one or more data items according to various exemplary embodiments of the present disclosure;
FIGS. 13A, 13B, 13C, and 13D illustrate examples of guide information according to various exemplary embodiments of the present disclosure;
FIGS. 14A and 14B illustrate examples of guide information according to various exemplary embodiments of the present disclosure;
FIGS. 15A and 15B illustrate examples of guide information according to various exemplary embodiments of the present disclosure; and
FIGS. 16A and 16B illustrate examples of guide information according to various exemplary embodiments of the present disclosure. Outline of the disclosure

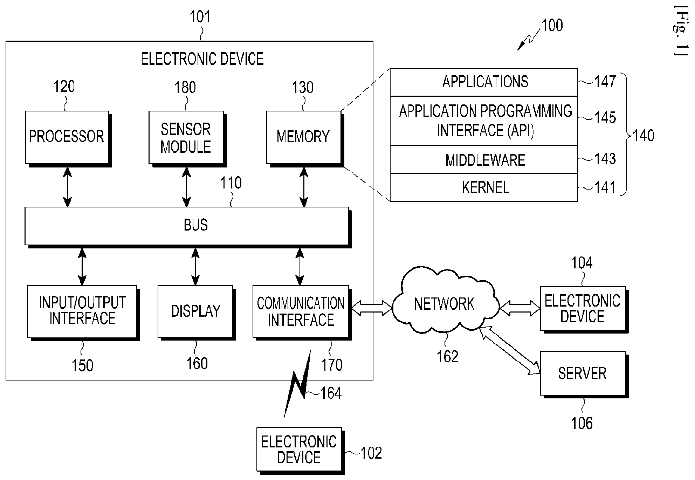

FIGS. 1 through 16B, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged system or device.

Hereinafter, various embodiments of the present disclosure will be described with reference to the accompanying drawings. The embodiments and the terms used therein are not intended to limit the technology disclosed herein to specific forms, and should be understood to include various modifications, equivalents, and/or alternatives to the corresponding embodiments. In the description of the drawings, similar reference numerals may be used to designate similar elements. A singular expression may include a plural expression unless they are definitely different in a context. In the present disclosure, the expression "A or B", "at least one of A and/or B", or "A/B" may include all possible combinations of the items listed. The expression "a first", "a second", "the first", or "the second" used in various embodiments of the present disclosure may modify various components regardless of the order and/or the importance but does not limit the corresponding components. When an element (e.g., first element) is referred to as being "(functionally or communicatively) connected," or "directly coupled" to another element (second element), the element may be connected directly to the another element or connected to the another element through yet another element (e.g., third element).

The expression "configured to" as used in various embodiments of the present disclosure may be interchangeably used with, for example, "suitable for", "having the capacity to", "designed to", "adapted to", "made to", or "capable of" in terms of hardware or software, according to circumstances. Alternatively, in some situations, the expression "device configured to" may mean that the device, together with other devices or components, "is able to". For example, the phrase "processor adapted (or configured) to perform A, B, and C" may mean a dedicated processor (e.g., embedded processor) only for performing the corresponding operations or a generic-purpose processor (e.g., Central Processing Unit (CPU) or Application Processor (AP)) that can perform the corresponding operations by executing one or more software programs stored in a memory device.

An electronic device according to various embodiments of the present disclosure may include at least one of, for example, a smart phone, a tablet Personal Computer (PC), a mobile phone, a video phone, an electronic book reader (e-book reader), a desktop PC, a laptop PC, a netbook computer, a workstation, a server, a Personal Digital Assistant (PDA), a Portable Multimedia Player (PMP), a MPEG-1 audio layer-3 (MP3) player, a mobile medical device, a camera, and a wearable device. According to various embodiments, the wearable device may include at least one of an accessory type (e.g., a watch, a ring, a bracelet, an anklet, a necklace, a glasses, a contact lens, or a Head-Mounted Device (HMD)), a fabric or clothing integrated type (e.g., an electronic clothing), a body-mounted type (e.g., a skin pad, or tattoo), and a bio-implantable type (e.g., an implantable circuit). In some embodiments, the electronic device may include at least one of, for example, a television, a Digital Video Disk (DVD) player, an audio, a refrigerator, an air conditioner, a vacuum cleaner, an oven, a microwave oven, a washing machine, an air cleaner, a set-top box, a home automation control panel, a security control panel, a TV box (e.g., SAMSUNG HOMESYNC, APPLE TV, or GOOGLE TV), a game console (e.g., XBOX and PLAYSTATION), an electronic dictionary, an electronic key, a camcorder, and an electronic photo frame.

In other embodiments, the electronic device may include at least one of various medical devices (e.g., various portable medical measuring devices (a blood glucose monitoring device, a heart rate monitoring device, a blood pressure measuring device, a body temperature measuring device, etc.), a Magnetic Resonance Angiography (MRA), a Magnetic Resonance Imaging (MRI), a Computed Tomography (CT) machine, and an ultrasonic machine), a navigation device, a Global Positioning System (GPS) receiver, an Event Data Recorder (EDR) , a Flight Data Recorder (FDR) , a Vehicle Infotainment Devices, an electronic devices for a ship (e.g., a navigation device for a ship, and a gyro-compass), avionics, security devices, an automotive head unit, a robot for home or industry, an automatic teller's machine (ATM) in banks, point of sales (POS) in a shop, or internet device of things (e.g., a light bulb, various sensors, electric or gas meter, a sprinkler device, a fire alarm, a thermostat, a streetlamp, a toaster, a sporting goods, a hot water tank, a heater, a boiler, etc.). According to some embodiments, an electronic device may include at least one of a part of furniture or a building/structure, an electronic board, an electronic signature receiving device, a projector, and various types of measuring instruments (e.g., a water meter, an electric meter, a gas meter, a radio wave meter, and the like). In various embodiments, the electronic device may be flexible, or may be a combination of one or more of the aforementioned various devices. The electronic device according to one embodiment of the present disclosure is not limited to the above described devices. In the present disclosure, the term "user" may indicate a person using an electronic device or a device (e.g., an artificial intelligence electronic device) using an electronic device.

FIG. 1 illustrates an electronic device 101 in a network environment 100 according to various exemplary embodiments. The electronic device 101 may include a bus 110, a processor 120, a memory 130, an input/output interface 150, a display 160, a communication interface 170, and a sensor module 180. In some exemplary embodiments, at least one of the components may be omitted, or an additional component may be further included in the electronic device 101.

The bus 110 may include a circuit that connects the components 120 to 180 to each other and delivers communications (for example, control messages or data) between the components.

The processor 120 may include one or more of a central processing unit, an application processor, and a communication processor (CP). The processor 120 may control, for example, at least one different component of the electronic device 101, and/or may perform an operation relating to communication or data processing.

According to one exemplary embodiment, the processor 120 may obtain status information corresponding to a user's activity or state associated with at least one data item of one or more health-related data items, may obtain user's genetic information, may select at least one piece of attribute information on the user's activity or state based on the obtained generic information, and may provide guide information related to the user's activity or state based on the at least one selected piece of attribute information.

The memory 130 may include a volatile and/or nonvolatile memory. The memory 130 may store, for example, a command or data related to at least one different component of the electronic device 101.

According to one exemplary embodiment, the memory 130 may store software and/ or a program 140. The program 140 may include, for example, a kernel 141, middleware 143, an Application Programming Interface (API) 145, and/or an application (or "application program ") 147. At least part of the kernel 141, the middleware 143, and the API 145 may be designated as an operating system.

The kernel 141 may control or manage system resources (for example, the bus 110, the processor 120, the memory 130, or the like) used to perform an operation or function implemented in other programs (for example, the middleware 143, the API 145, or the application 147). Further, the kernel 141 may provide an interface that allows the middleware 143, the API 145, or the application 147 to access individual components of the electronic device 101 to thereby control or manage system resources.

The middleware 143 may serve as a relay so that, for example, the API 145 or the application 147 communicates with the kernel 141 to exchange data. Further, the middleware 143 may process one or more requests for operations received from the application 147 according to priority. For example, the middleware 143 may assign at least one application 147 a priority for using a system resource (for example, the bus 110, the processor 120, the memory 130, or the like) of the electronic device 101, and may process the one or more requests for operations. The API 145 is an interface for the application 147 to control a function provided from the kernel 141 or the middleware 143, and may include, for example, at least one interface or function (for example, a command) for file control, window control, image processing, or text control. The input/output interface 150 may deliver a command or data, which is input from, for example, a user or a different external device, to a different component(s) of the electronic device 101 or may output a command or data, which is received from a different component(s) of the electronic device 101, to the user or different external device.

The display 160 may include, for example, a Liquid Crystal Display (LCD), a Light-Emitting Diode (LED) display, an Organic Light-Emitting Diode (OLED) display, a Micro-Electro-Mechanical Systems (MEMS) display, and an electronic paper display. The display 160 may display, for example, various types of content (for example, a text, an image, a video, an icon, a symbol, and/or the like) for the user. According to various exemplary embodiments, the display 160 may include a touch screen and may receive touch, gesture, proximity, drag, swipe, or hovering input using, for example, an electronic pen or a body part of a user.

The communication interface 170 may establish communication, for example, between the electronic device 101 and an external device (for example, a first external electronic device 102, a second external electronic device 104, or a server 106). For example, the communication interface 170 may be connected to a network 162 via wireless communication or wire-based communication to communicate with the external device (for example, the second external electronic device 104 or the server 106).

The wireless communication may include cellular communication using, for example, at least one of Long-Term Evolution (LTE), LTE-Advanced (LTE-A), code division multiple access (CDMA), wideband CDMA (WCDMA), universal mobile telecommunications system (UMTS), Wireless Broadband (WiBro), and Global System for Mobile Communications (GSM). In one exemplary embodiment, the wireless communication may include, for example, at least one of Wireless Fidelity (WI-FI), BLUETOOTH, Light Fidelity (Li-Fi), BLUETOOTH Low Energy (BLE), ZIGBEE, Near Field Communication (NFC), Magnetic Secure Transmission, Radio Frequency (RF), and a Body Area Network (BAN), as indicated by 164 in FIG. 1. In one exemplary embodiment, the wireless communication may include a Global Navigation Satellite System (GNSS). The GNSS may be, for example, a Global Positioning System (GPS), a global navigation satellite system (Glonass), a Beidou navigation satellite system (hereinafter, "Beidou"), or Galileo, which is the European global satellite-based navigation system. In the present document, "GPS" may be interchangeably used with "GNSS" hereinafter. The wire-based communication may include, for example, at least one of Universal Serial Bus (USB), High Definition Multimedia Interface (HDMI), Recommended Standard 232 (RS-232), Power Line Communication, and Plain Old Telephone Service (POTS). The network 162 may include a telecommunications network, which may be, for example, at least one of a computer network (for example, a Local Area Network (LAN) or Wide Area Network (WAN)), the Internet, and a telephone network.

The sensor module 180 may include an accelerometer, a pedometer sensor, a gyro sensor, a heart rate sensor, or the like and may obtain status information corresponding to a user's activity or state.

The first and second external electronic devices 102 and 104 may each be a device of a type that is the same as, or different from, the electronic device 101.

According to various exemplary embodiments, all or part of the operations performed in the electronic device 101 may be performed in another electronic device or a plurality of electronic devices (for example, the electronic devices 102 and 104 or the server 106).

According to one exemplary embodiment, when the electronic device 101 needs to perform a function or service automatically or upon request, the electronic device 101 may request another electronic device (for example, the electronic device 102 or 104, or the server 106) to perform at least some functions related to the function or service, instead of, or in addition to, autonomously performing the function or service. The other electronic device (for example, the electronic device 102 or 104, or the server 106) may perform the requested functions or additional function and may transmit the result thereof to the electronic device 101. The electronic device 101 may provide the requested function or service using the same received result or by additionally processing the result. To this end, cloud computing, distributed computing, or client-server computing technologies may be used.

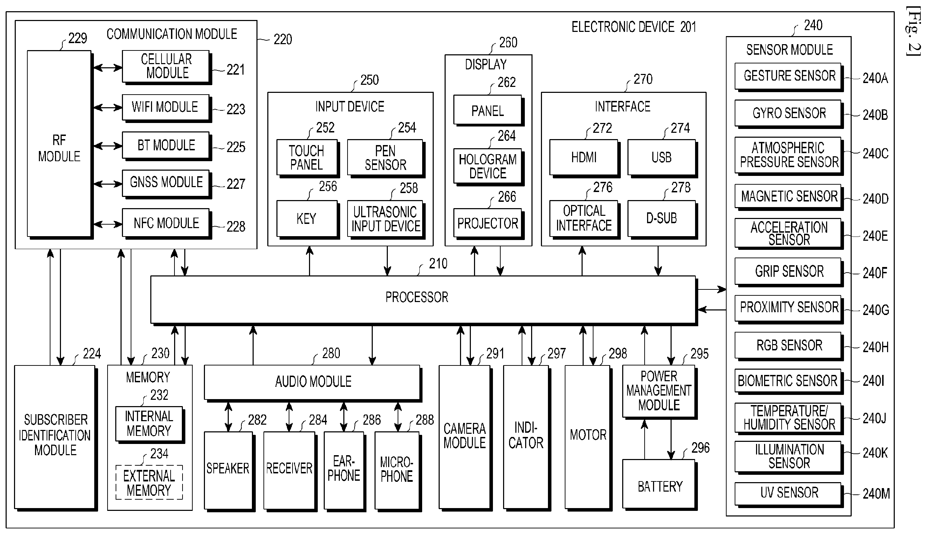

FIG. 2 is a block diagram of an electronic device 201 according to various exemplary embodiments. The electronic device 201 may include, for example, all or part of the electronic device 101 illustrated in FIG. 1. The electronic device 201 may include one or more processors 210, a communication module 220, a Subscriber Identification Module (SIM) 224, a memory 230, a sensor module 240, an input device 250, a display 260, an interface 270, an audio module 280, a camera module 291, a power management module 295, a battery 296, an indicator 297, and a motor 298. The processors 210 may run, for example, an operating system or an application to control a plurality of hardware or software components that are connected to the processors 210 and may perform various kinds of data processing and operations. The processors 210 may be configured, for example, as a System on Chip (SoC).

According to one exemplary embodiment, the processors 210 may further include a Graphic Processing Unit (GPU) and/or an image signal processor. The processors 210 may include at least part (for example, a cellular module 221) of the components illustrated in FIG. 2. The processors 210 may load a command or data received from at least one of other components (for example, a nonvolatile memory) into a volatile memory to process the command or data, and may store resulting data in the nonvolatile memory.

According to one exemplary embodiment, the processors 210 may obtain status information corresponding to a user's activity or state associated with at least one data item of one or more health-related data items, may obtain user's genetic information, may select at least one piece of attribute information on the user's activity or state based on the obtained generic information, and may provide guide information related to the user's activity or state based on the at least one selected piece of attribute information.

The communication module 220 may have a configuration that is the same as, or similar to, that of the communication interface 170. The communication module 220 may include, for example, a cellular module 221, a Wi-Fi module 223, a Bluetooth module 225, a GNSS module 227, an NFC module 228, and an RF module 229. The cellular module 221 may provide, for example, a voice call, a video call, a text messaging service, or an Internet service through a communication network. According to one exemplary embodiment, the cellular module 221 may perform identification and authentication of the electronic device 201 in a communication network using a Subscriber Identity Module (SIM, for example, a SIM card) 224. According to one exemplary embodiment, the cellular module 221 may perform at least part of the functions provided by the processors 210. According to one exemplary embodiment, the cellular module 221 may include a Communication Processor (CP). According to one exemplary embodiment, at least some (for example, two or more) of the cellular module 221, the Wi-Fi module 223, the Bluetooth module 225, the GNSS module 227, and the NFC module 228 may be included in one Integrated Chip (IC) or IC package. The RF module 229 may transmit and receive, for example, a communication signal (for example, an RF signal). The RF module 229 may include, for example, a transceiver, a Power Amplifier (amp) Module (PAM), a frequency filter, a Low Noise Amplifier (LNA), at least one antenna, or the like. According to another exemplary embodiment, at least one of the cellular module 221, the Wi-Fi module 223, the Bluetooth module 225, the GNSS module 227, and the NFC module 228 may transmit and receive an RF signal through a separate RF module. The SIM 224 may include, for example, a card including an SIM or an embedded SIM and may include unique identification information (for example, an Integrated Circuit Card Identifier (ICCID)) or subscriber information (for example, an International Mobile Subscriber Identity (IMSI)).

The memory 230 (for example, the memory 130) may include, for example, an internal memory 232 or an external memory 234. The internal memory 232 may include, for example, at least one of a volatile memory (for example, a DRAM, an SRAM, an SDRAM, or the like) and a nonvolatile memory (for example, an OTPROM, a PROM, an EPROM, an EEPROM, a mask ROM, a flash ROM, a flash memory, a hard drive, or a Solid State Drive (SSD)). The external memory 234 may include a flash drive, for example, a Compact Flash (CF), a Secure Digital (SD), a Micro-SD, a Mini-SD, an extreme digital (xD), a Multi-Media Card (MMC), a memory stick, or the like. The external memory 234 may be functionally or physically connected to the electronic device 201 through various interfaces.

The sensor module 240 may measure, for example, physical quantities, or may detect the state of operation of the electronic device 201 and convert measured or detected information into an electrical signal. The sensor module 240 may include, for example, at least one of a gesture sensor 240A, a gyro sensor 240B, a barometric pressure sensor 240C, a magnetic sensor 240D, an accelerometer 240E, a grip sensor 240F, a proximity sensor 240G, a color sensor 240H (for example, a red, green, and blue (RGB) sensor), a biometric sensor 240I, a temperature/humidity sensor 240J, an illumination sensor 240K, and an ultraviolet (UV) sensor 240M. Additionally or alternatively, the sensor module 240 may include, for example, an E-nose sensor, an electromyography (EMG) sensor, an electroencephalogram (EEG) sensor, an electrocardiogram (ECG) sensor, an infrared (IR) sensor, an iris sensor, and/or a fingerprint sensor. The sensor module 240 may further include a control circuit to control at least one or more sensors belonging thereto. In one exemplary embodiment, the electronic device 201 may further include a processor configured, as part of the processors 210 or separately from the processors 210, to control the sensor module 240, thereby controlling the sensor module 240 while the processors 210 are in a sleep state.

The input device 250 may include, for example, a touch panel 252, a (digital) pen sensor 254, a key 256, or an ultrasonic input device 258. The touch panel 252 may be, for example, at least one of an electrostatic type, a pressure-sensitive type, an infrared type, and an ultrasonic type. Further, the touch panel 252 may further include a control circuit. The touch panel 252 may further include a tactile layer to provide a user with a tactile response. The (digital) pen sensor 254 may, for example, be part of the touch panel or may include a separate recognition sheet. The key 256 may include, for example, a physical button, an optical key, or a keypad. The ultrasonic input device 258 may detect ultrasonic waves generated in an input tool through a microphone (for example, a microphone 288) and may identify data corresponding to the detected ultrasonic waves.

The display 260 (for example, the display 160) may include a panel 262, a hologram device 264, a projector 266, and/or a control circuit to control the panel 262, the hologram device 264, or the projector 266. The panel 262 may be configured, for example, to be flexible, transparent, or wearable. The panel 262 may be formed with the touch panel 252 in one or more modules. According to one exemplary embodiment, the panel 262 may include a pressure sensor (or force sensor) to measure the strength of pressure of a user's touch. The pressure sensor may be formed with the touch panel 252 in a single body, or may be provided as one or more sensors separate from the touch panel 252. The hologram device 264 may display a three-dimensional image in the air using the interference of light. The projector 266 may project light onto a screen to display an image. The screen may be disposed, for example, inside or outside the electronic device 201. The interface 270 may include, for example, an HDMI 272, a USB 274, an optical interface 276, or a D-subminiature (D-sub) interface 278. The interface 270 may be included, for example, in the communication interface 170 illustrated in FIG. 1. Additionally or alternatively, the interface 270 may include, for example, a Mobile High-definition Link (MHL) interface, an SD card/MMC interface, or an Infrared Data Association (IrDA) interface.

The audio module 280 may bi-directionally convert, for example, a sound and an electrical signal. At least some components of the audio module 280 may be included, for example, in the input/output interface 150 illustrated in FIG. 1. The audio module 280 may process sound information input or output, for example, through a speaker 282, a receiver 284, earphones 286, or the microphone 288.

The camera module 291 is a device that takes, for example, a still image and a video. According to one exemplary embodiment, the camera module 291 may include one or more image sensors (for example, a front sensor or a rear sensor), a lens, an Image Signal Processor (ISP), or a flash (for example, an LED, a xenon lamp, or the like).

The power management module 295 may manage, for example, the power of the electronic device 201. According to one exemplary embodiment, the power management module 295 may include a Power Management Integrated Circuit (PMIC), a charger Integrated Circuit (IC), or a battery or fuel gauge. The PMIC may have wired and/or wireless charging methods. The wireless charging methods may include, for example, a magnetic-resonance method, a magnetic-induction method, or an electromagnetic-wave method, and may further include an additional circuit for wireless charging, such as a coil loop, a resonance circuit, or a rectifier. The battery gauge may measure, for example, the remaining battery charge, the charging voltage, the current, or the temperature of the battery 296. The battery 296 may include, for example, a rechargeable battery and/or a solar battery.

The indicator 297 may display the specific state of the electronic device 201 or a component thereof (for example, the processors 210), which may be, for example, a booting state, a message state, or a charging state. The motor 298 may convert an electrical signal into mechanical vibrations and may generate vibrations or a haptic effect. The electronic device 201 may include a mobile TV support device (for example, a GPU) that is capable of processing media data in accordance with, for example, Digital Multimedia Broadcasting (DMB), Digital Video Broadcasting (DVB), or MEDIAFLO standards. Each element mentioned in the present document may include one or more components, and may be designated by different terms depending on the type of the electronic device. In various exemplary embodiments, an electronic device (for example, the electronic device 201) may be configured such that some elements are omitted, additional elements are further included, or some of the elements are combined into one entity, but may perform the same functions as those of the corresponding elements before combination.

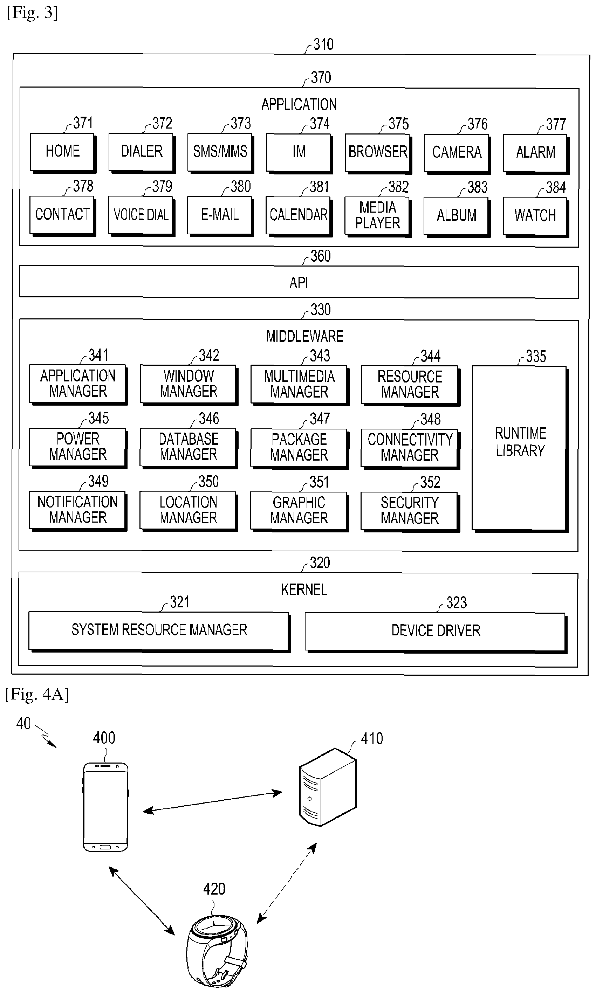

FIG. 3 is a block diagram of a program module according to various exemplary embodiments. According to one exemplary embodiment, the program module 310 (for example, the program 140) may include an operating system that controls resources related to an electronic device (for example, the electronic device 101) and/or various applications (for example, the application 147) that run on the operating system. The operating system may include, for example, ANDROID, IOS, WINDOWS, SYMBIAN, TIZEN, BADA, or the like. Referring to FIG. 3, the program module 310 may include a kernel 320 (for example, the kernel 141), middleware 330 (for example, the middleware 143), an API 360 (for example, the API 145), and/or an application 370 (for example, the application 147). At least part of the program module 310 may be preloaded onto the electronic device or may be downloaded from an external electronic device (for example, the electronic device 102 or 104, the server 106, or the like).

The kernel 320 may include, for example, a system resource manager 321 and/or a device driver 323. The system resource manager 321 may perform control, allocation, or recovery of system resources. According to one exemplary embodiment, the system resource manager 321 may include a process manager, a memory manager, or a file system manager. The device driver 323 may include, for example, a display driver, a camera driver, a BLUETOOTH driver, a shared memory driver, a USB driver, a keypad driver, a WI-FI driver, an audio driver, or an Inter-Process Communication (IPC) driver. The middleware 330 may provide, for example, functions commonly needed for applications 370, or may provide an application 370 with various functions through the API 360 so that the application 370 may use the limited systems resources in the electronic device. According to one exemplary embodiment, the middleware 330 may include at least one of a runtime library 335, an application manager 341, a window manager 342, a multimedia manager 343, a resource manager 344, a power manager 345, a database manager 346, a package manager 347, a connectivity manager 348, a notification manager 349, a location manager 350, a graphic manager 351, and a security manager 352.

The runtime library 335 may include, for example, a library module used by a complier to add a new function through a programming language while the application 370 is running. The runtime library 335 may perform input/output management, memory management, or arithmetic function processing. The application manager 341 may manage, for example, the life cycle of the application 370. The window manager 342 may manage Graphic User Interface (GUI) resources used for a screen. The multimedia manager 343 may identify formats that are necessary to play media files, and may encrypt or decrypt a media file using a codec suitable for a corresponding format. The resource manager 344 may manage a source code or memory space for the application 370. The power manager 345 may manage battery capacity, temperature, or power supply, and may determine or provide information on power necessary for the operation of the electronic device using corresponding information among these. According to one exemplary embodiment, the power manager 345 may interwork with a Basic Input/Output System (BIOS). The database manager 346 may generate, retrieve, or change a database to be used for, for example, the application 370. The package manager 347 may install or update an application distributed in the form of a package file.

The connectivity manager 348 may manage, for example, wireless connectivity. The notification manager 349 may provide a user with an event, for example, an incoming message, an appointment, and a proximity notification. The location manager 350 may manage, for example, information about the location of the electronic device. The graphic manager 351 may manage, for example, a graphic effect to be provided for the user or a user interface related to the graphic effect. The security manager 352 may provide, for example, system security or user authentication. According to one exemplary embodiment, the middleware 330 may include a telephony manager to manage a voice or video call function of the electronic device or a middleware module that is capable of forming combinations of functions of the foregoing elements. According to one exemplary embodiment, the middleware 330 may provide a specialized module for each operating system. The middleware 330 may dynamically delete some of the existing elements or add new elements. The API 360 is, for example, a set of API programming functions, and may be provided with a different configuration depending on the operating system. For example, one API set for each platform may be provided in Android or iOS, while two or more API sets for each platform may be provided in Tizen.

The application 370 may include, for example, a home 371, a dialer 372, an SMS/ MMS 373, an Instant Message (IM) 374, a browser 375, a camera 376, an alarm 377, a contact 378, a voice dialer 379, an email 380, a calendar 381, a media player 382, an album 383, a watch 384, a healthcare application (for example, for measuring exercising or blood sugar), or an application providing environmental data (for example, atmospheric pressure, humidity, or temperature data). According to one exemplary embodiment, the application 370 may include an information exchange application that is capable of supporting information exchange between the electronic device and an external electronic device. The information exchange application may include, for example, a notification relay application for relaying specific information to the external electronic device or a device management application for managing the external electronic device. For example, the notification relay application may relay notification information, which is generated in another application of the electronic device, to the external electronic device, or may receive notification information from the external electronic device and provide the notification information to the user. The device management application may install, delete, or update, for example, a function

(for example, a function of turning on/turning off the external electronic device itself (or some components) or adjusting the brightness (or resolution) of a display) of an external electronic device communicating with the electronic device or an application operating in the external electronic device. According to one exemplary embodiment, the application 370 may include an application (for example, a healthcare application of a mobile medical device) assigned according to the attributes of the external electronic device. According to one exemplary embodiment, the application 370 may include an application received from the external electronic device.

At least part of the program module 310 may be implemented (for example, run) by software, firmware, hardware (for example, the processors 210), or combinations of at least two or more thereof, and may include a module, a program, a routine, sets of instructions, or a process to perform one or more functions.

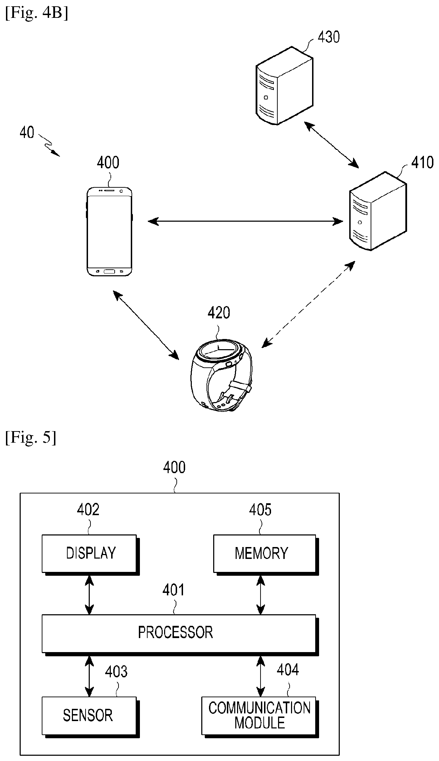

FIGS. 4A and 4B illustrate configurations of a system according to various exemplary embodiments.

Referring to FIG. 4A, the system 40 may include an electronic device 400 (for example, the electronic device 101 or 201), a first server 410 (for example, the server 106), and an external electronic device 420 (for example, the electronic device 102 or 104). According to various exemplary embodiments, the external electronic device 420 may optionally be included.

According to one exemplary embodiment, the electronic device 400 may display, on a display, a health-related user interface based on genetic information on a user and status information corresponding to an activity or state of the user. The activity of the user may include the user's actions, such as eating food, drinking water, drinking coffee with caffeine, exercising including walking, running, swimming, cycling and hiking, and sleeping. The state of the user may include at least one of body information on the user's body, such as age, height, weight and waist size, and biometric information, such as heart rate, oxygen saturation level, blood glucose level, blood pressure level and stress index. The user interface may include one or more health-related data items, and each of the data items may include a graphic object (for example, a text, an image, an icon, and a widget) corresponding to an activity or state of the user. For example, the user interface may be an execution screen of a health-related application (for example, S Health) that provides health-related information.

According to one exemplary embodiment, the electronic device 400 may obtain status information corresponding to an activity or state of the user associated with at least one of the one or more data items. For example, the status information may include at least one of height, weight, heart rate, oxygen saturation level, stress index, blood glucose level, blood pressure level, sleeping time/degree, the time/ duration/degree of exposure to ultraviolet rays, a step count, walking time/ duration/degree, running time/duration/degree, cycling time/duration/degree, hiking time/duration/degree, the time/type/duration/degree of a sport that the user played, the kind of food the user ate, time/amount/degree regarding food intake, time/ amount/degree regarding water drinking, and time/amount/degree regarding caffeine intake. For example, when a first input for running the health-related application is received, the electronic device 400 may obtain status information (for example, a step count, heart rate, oxygen saturation level, blood glucose level, blood pressure level, and stress index) corresponding to an activity or state of the user using a sensor (for example, an accelerometer, a pedometer sensor, a gyro sensor, and a heart rate sensor) provided in the electronic device 400. Further, the electronic device 400 may receive an input related to an activity or state of the user and may obtain status information (for example, the type/intake time/intake amount of food, the intake time/intake amount of water, the intake time/intake amount of caffeine, weight, and sleeping time). Also, the electronic device 400 may analyze usage information on the electronic device 400 (for example, application usage information, payment details information, search information, and schedule information) and may obtain status information corresponding to an analysis result (for example, the type/intake time/intake amount of food, the intake time/intake amount of water, the intake time/intake amount of caffeine, the time/number of outdoor activities, and whether the user receives scalp treatments).

According to various exemplary embodiments, when the electronic device 400 has no sensor or can obtain some status information using a sensor, the electronic device 400 may request at least some status information from another electronic device. For example, the electronic device 400 may transmit a signal for requesting at least some status information to the external electronic device 420 and may receive at least some status information from the external electronic device 420. For example, the external electronic device 420 may measure the user's heart rate, blood sugar level, blood pressure level, stress index, a step count, and the like using a sensor provided in the external electronic device 420 and may provide at least some status information corresponding to a measurement result.

According to one exemplary embodiment, the electronic device 400 may obtain genetic information on the user. The electronic device 400 may store the genetic information on the user in a Security Enhancement (SE) area of a memory or may request and receive the genetic information from a first server 410 that provides the genetic information on the user.

According to one exemplary embodiment, the genetic information may be raw data including a result of analyzing tissues collected from the user's tongue, hair, or the like.

According to one exemplary embodiment, the genetic information may include at least some information extracted corresponding to at least one data item of the raw data.

According to one exemplary embodiment, the genetic information may include data obtained by processing the raw data according to a certain criterion. For example, the genetic information may include information on whether the user has a genotype of low caffeine metabolism or has a genotype of a high probability of hair loss.

According to various exemplary embodiments, the genetic information may be raw data or processed data including results of analyzing tissues collected from the tongue or hair of the user, and can also include a person related to the user by blood (for example, a family member).

According to one exemplary embodiment, the electronic device 400 may select attribute information on an activity or state of the user based on at least some of the obtained genetic information. The attribute information may include at least one of priority information on one or more data items, accumulated numerical information corresponding to an activity or statue of the user, and desired numerical information. According to various exemplary embodiments, the attribute information may be calculated based on the genetic information and the status information.

According to one exemplary embodiment, the priority information may include information on the ranking of each data item calculated based on the genetic information on the user and the status information. For example, the electronic device 400 may analyze the genetic information and the status information, and may set the ranking of a caffeine-related data item to be higher than the rankings of other data items when determining that the user has genes lacking an enzyme involved in caffeine degradation and the user's daily caffeine intake is higher than the average caffeine intake. The information on the rankings thus set may be the priority information.

According to various exemplary embodiments, the priority information may include information on the rankings of respective data items set according to the degree (or level) of risk of a corresponding disease or physical constitution based on the genetic information on the user. For example, the ranking of a disease-related data item may be higher than that of a physical constitution-related data item, and the ranking of a data item related to a disease or physical constitution with a high risk level may be higher than that of a data item related to a disease or physical constitution with a low risk level.

According to one exemplary embodiment, the accumulated numerical information may include information obtained by quantifying the amount of data accumulated for each data item based on the status information. For example, accumulated numerical information corresponding to a caffeine-related data item may include user's daily caffeine intake (for example, the number of times the user drank coffee/amount of coffee the user drank). For example, accumulated numerical information corresponding to a walking-related data item may include a step count, time, and/or distance with respect to user's action of walking, which are obtained using a sensor. The accumulated numerical information may be periodically (or continuously) updated based on at least some of the obtained status information.

According to one exemplary embodiment, the desired numerical information may be information obtained by quantifying a desired data amount determined for each data item based on the genetic information, the status information, and rule information set corresponding to the activity or state of the user. For example, desired numerical information corresponding to a caffeine-related data item may include a daily caffeine intake (for example, one cup a day) recommended to the user. For example, desired numerical information corresponding to a walking-related data item may include walking time, a step count, and/or walking distance recommended per day to the user.

According to various exemplary embodiments, the desired numerical information may be set based on medical data, articles, research results, and the like.

According to various exemplary embodiments, the desired numerical information may be set or recommended based on information proposed by an expert, such as a doctor, a fitness instructor, or the like. For example, the information proposed by the expert may include exercise, a diet, or an advice.

According to various exemplary embodiments, the desired numerical information may be set based on information on other users having similar genetic information by analyzing health big data. For example, when another user with genes lacking an enzyme involved in caffeine degradation sets caffeine intake to three cups of coffee a day, three cups of coffee a day may be set or recommended for the desired numerical information. The information on the other users may be stored in the memory of the first server 410.

According to various exemplary embodiments, the desired numerical information may be set by the user.

According to various exemplary embodiments, the desired numerical information may be automatically set and may be reset by the user checking.

According to one exemplary embodiment, the electronic device 400 may display, on the display, guide information related to the activity or state of the user based on the selected attribute information.

According to one exemplary embodiment, the guide information may include one or more data items arranged based on the priority information. For example, the one or more data items may include a graphical object corresponding to the activity or state of the user as well as a graphical object corresponding to healthcare information associated with the activity or state of the user. The healthcare information may include various pieces of health-related information corresponding to the activity or the state of the user.

The electronic device 400 may calculate priority information on one or more data items based on the genetic information and the status information and may determine an output attribute (for example, output position, output size, and output color) of each output data item based on the calculated priority information. The electronic device 400 may display one or more data items on the display based on the determined output attribute.

According to various exemplary embodiments, the electronic device 400 may display a first-ranked data item at a first position (for example, at the top of a display area for displaying a data item) based on an output attribute (for example, position) and may display a second-ranked data item at a second position (for example, below or at the right of the first-ranked data item) different from the first position. For example, the one or more data items may be displayed in order from the highest-ranked data item to the lowest-ranked data item.

According to various exemplary embodiments, the electronic device 400 may display the first-ranked data item corresponding to the highest ranking in a first size based on an output attribute (for example, size) and may display the second-ranked data item corresponding to the next highest ranking in a second size smaller than the first size. The electronic device 400 may display the lowest-ranked item in the smallest size.

According to various exemplary embodiments, the electronic device 400 may display at least a portion of the highest-ranked data item in a first color (for example, red) based on an output attribute (for example, color) and may display at least a portion of the lowest-ranked data item in a second color (for example, blue).

According to various exemplary embodiments, when the activity or state of the user changes, the electronic device 400 may determine priority information based on status information corresponding to the changed activity or state and may display data items by adding, deleting, or rearranging at least one data item based on the determined priority information.

According to one exemplary embodiment, the guide information may include a notification associated with the activity or state of the user based on the accumulated numerical information and the desired numerical information.

For example, the electronic device 400 may obtain status information, such as the amount of coffee the user purchased in one day, the time when the user purchased coffee, or whether the user added shots of espresso, and may obtain accumulated numerical information based on the obtained status information. When the amount of coffee the user drank in one day (for example, the daily intake of coffee) is three cups and the user added shots of espresso, accumulated numerical information corresponding to caffeine (or caffeine intake) may be about 100 to 150 mg. The accumulated numerical information may be continuously updated whenever the user purchases caffeinated coffee or a beverage or by user's input.

The electronic device 400 may determine desired numerical information corresponding to caffeine based on genetic information on the user corresponding to caffeine, status information, such as time/number of times/amount/kind/shot addition relative to caffeinated coffee or beverage the user drinks, and rule information corresponding to caffeine. The rule information may include a rule that limits caffeine intake, for example, based on the recommended daily intake of caffeine for adults (for example, 300 mg/day) with respect to caffeinated coffee or beverage.

When the user is determined to have genes lacking an enzyme involved in caffeine degradation, the electronic device 400 may determine a caffeine intake of 300 mg per day as the desired numerical information based on the genetic information, the status information, and the rule information.

According to various exemplary embodiments, the electronic device 400 may automatically set a limit value relative to caffeine intake (for example, the number of cups of coffee) when the user is determined to have genes lacking an enzyme involved in caffeine degradation. For example, the electronic device 400 may automatically set "three cups of coffee a day" as the limit value.

The electronic device 400 may compare the accumulated numerical information with the desired numerical information to determine whether a comparison result satisfies a specified condition. When the comparison result satisfies the specified condition, the electronic device 400 may display a notification associated with the activity or state of the user on the display. For example, the specified condition may include a case where the daily caffeine intake is greater than or equal to the recommended daily intake of caffeine for adults, a case where the difference between the daily caffeine intake and the recommended daily intake of caffeine for adults is less than a threshold, and a case where the daily caffeine intake does not exceed the recommended daily intake of caffeine for adults but the monthly caffeine intake exceeds the recommended monthly intake of caffeine.

When the comparison result satisfies the specified condition, the electronic device 400 may provide a notification to report that the daily caffeine intake of the user is greater than or equal to the recommended daily intake of caffeine for adults.

According to various exemplary embodiments, the electronic device 400 may provide a notification in order to report that the daily caffeine intake comes close to the recommended daily intake for adults or to report that the daily caffeine intake does not exceed the recommended daily intake of caffeine but the monthly caffeine intake is greater than or equal to the recommended monthly intake of caffeine.

According to various exemplary embodiments, the electronic device 400 may not provide a caffeine-related notification when the daily caffeine intake exceeds the recommended daily intake of caffeine for adults but the monthly caffeine intake does not exceed the recommended monthly intake of caffeine.

According to various exemplary embodiments, when the comparison result satisfies the specified condition, the electronic device 400 may provide information for advising a decrease in caffeine intake or notification information for reporting the recommended daily intake of caffeine for adults.

According to various exemplary embodiments, when the comparison result satisfies the specified condition, the electronic device 400 may sense a change in state, such as sleeping time, heart rate, time to fall asleep, sleep quality, stress level, or fatigue level, by caffeine intake, and may provide a notification for reporting a state change when the sleeping time, heart rate, time to fall asleep, sleep quality, stress level or fatigue level is changed by a threshold or greater.

According to various exemplary embodiments, the electronic device 400 may provide information for limiting caffeine intake after a specified time based on caffeine-related status information on the user. For example, the electronic device 400 may analyze user's sleeping time, heart rate, time to fall asleep, sleep quality, stress level or fatigue level, and may provide notification information for limiting caffeine intake after a specified time when it is determined that the user has a problem with sleeping.

According to various exemplary embodiments, when the comparison result satisfies the specified condition, the electronic device 400 may reset the desired numerical information based on the activity or state of the user. For example, when the desired numerical information corresponding to caffeine is set to "two cups of coffee per day" based on the genetic information, if it is determined that the user drinks two cups of coffee a day or the user drinks two cups of coffee for consecutive days, the electronic device 400 may check the state of the user (for example, sleeping time, heart rate, time to fall asleep, sleep quality, stress level or fatigue level) based on the status information and may reset the desired numerical information based on the checked state. When it is determined that two cups of coffee do not affect sleeping time, heart rate, or the like, the electronic device 400 may reset the desired numerical information to "three cups of coffee per day."

According to various exemplary embodiments, the electronic device 400 may obtain the genetic information on the user, may determine the priority of at least one data item among one or more heal-related data items based on the obtained genetic information, and may provide at least one data item based on the determined priority. The electronic device 400 may analyze the obtained genetic information and may calculate the ranking of each data item according to the degree of risk of a disease or physical constitution related to each data item. For example, a data item related to a disease or physical constitution with a high risk level may have a higher ranking, and a data item related to a disease or physical constitution with a low risk level may have a lower ranking. The electronic device 400 may provide at least one data item in order from a higher-ranked data item to a lower-ranked data item.

According to various exemplary embodiments, the electronic device 400 may transmit the status information to the first server 410 upon request and may receive guide information associated with the activity or state of the user from the first server 410.

According to various exemplary embodiments, the electronic device 400 may transmit the status information to the first server 410 upon request and may receive at least one of priority information, rule information, and desired numerical information from the first server 410.

According to various exemplary embodiments, the electronic device 400 may transmit the genetic information on the user and status information to the first server 410 upon request and may receive at least one of guide information, priority information, rule information, and desired numerical information from the first server 410.

According to one exemplary embodiment, the first server 410 may obtain genetic information on the user and may provide the obtained genetic information to the electronic device 400 or the external electronic device 420. The first server 410 may analyze tissues collected from the user's tongue or hair and may store genetic information including an analysis result in a Security Enhancement (SE) area of a memory. For example, the first server 410 may receive a request for genetic information from the electronic device 400 or external electronic device 420 and may transmit the stored genetic information to the electronic device 400 or the external electronic device 420 in response to the request.

According to various exemplary embodiments, the first server 410 may transmit raw data including the analysis result, or may extract and transmit at least a portion of the raw data corresponding to at least one health object associated with health.

According to various exemplary embodiments, the first server 410 may store one or more health-related data items in the memory.

According to various exemplary embodiments, the first server 410 may receive and store one or more data items and status information corresponding to an activity or state of the user from the electronic device 400 or the external electronic device 420 and may select attribute information on the activity or state of the user based on at least some of the genetic information. The first server 410 may transmit the selected attribute information to the electronic device 400 or the external electronic device 420. The attribute information may include at least one of priority information, accumulated numerical information, and desired numerical information. For example, the first server 410 may provide at least one of the priority information, the accumulated numerical information, and the desired numerical information upon request from the electronic device 400 or the external electronic device 420.

According to various exemplary embodiments, the first server 410 may transmit guide information associated with the activity or state of the user to the electronic device 400 or the external electronic device 420 based on the attribute information.

According to various exemplary embodiments, when the attribute information is received from the electronic device 400 or the external electronic device 420, the first server 410 may transmit the guide information based on the received attribute information to the electronic device 400 or the external electronic device 420.

According to various exemplary embodiments, the first server 410 may determine the priority of at least one data item among one or more data items based on the genetic information and may provide information on the determined priority to the electronic device 400 or the external electronic device 420.

According to one exemplary embodiment, the external electronic device 420 may perform at least some operations that are the same as those of the electronic device 400. For example, the external electronic device 420 may be a wearable device (for example, a watch).

According to one exemplary embodiment, the external electronic device 420 may include a sensor and may obtain status information corresponding to an activity or state of the user through the sensor. The external electronic device 420 may transmit the obtained status information to the electronic device 400.

According to one exemplary embodiment, the external electronic device 420 may obtain status information associated with at least one data item among one or more health-related items and may obtain genetic information on the user. For example, the external electronic device 420 may request the genetic information from the first server 410 and may receive the genetic information from the first server 410 in response to the request. For example, the external electronic device 420 may request the genetic information from the electronic device 400 and may receive the genetic information from the electronic device 400 in response to the request. The external electronic device 420 may select attribute information on the activity or state of the user based on at least some of the obtained genetic information and may provide guide information associated with the activity or state based on the selected attribute information.

According to various exemplary embodiments, the external electronic device 420 may transmit the status information to the first server 410 upon request from the first server 410, and may receive at least one of priority information, rule information, and desired numerical information or may receive guide information from the first server 410. For example, the external electronic device 420 may provide guide information based on at least one of the priority information, the rule information, and the desired numerical information received from the first server 410 or may provide the guide information received from the first server 410.

According to various exemplary embodiments, the external electronic device 420 may determine the priority of at least one data item among one or more data items based on the genetic information and may provide information on the determined priority to the electronic device 400.

According to various exemplary embodiments, the external electronic device 420 may receive information on a priority from the electronic device 400 or the first server 410 and may provide at least one item based on the received information on the priority.

Referring to FIG. 4B, the system 40 may include an electronic device 400, a first server 410, an external electronic device 420, and a second server 430. According to various exemplary embodiments, the external electronic device 420 may be optionally included, and the second server 430 may be a server that provides genetic information on a user.

According to one exemplary embodiment, the electronic device 400 may perform at least some operations that are the same as those of the electronic device of FIG. 4A.

According to one exemplary embodiment, the electronic device 400 may request at least some of status information corresponding to an activity or state of the user from the external electronic device 420 and may receive at least some of the status information from the external electronic device 420 in response to the request.

According to one exemplary embodiment, the electronic device 400 may request genetic information on the user from the first server 410, and the first server 410 may generate a request signal for requesting the genetic information on the user in response to the request and may transmit the request signal to the second server 430.

Upon receiving the request signal, the second server 430 may transmit a response signal including the genetic information on the user to the first server 410. For example, the second server 430 may store data (for example, genetic information) corresponding to an experimental result, which is obtained by collecting tissues from the user's tongue or hair, analyzing the collected tissues to extract genetic data, and conducting an experiment on the extracted genetic data. The genetic information may be raw data or processed data corresponding to at least one health object.

Upon receiving a response signal including raw data, the first server 410 may transmit the raw data to the electronic device 400 or may transmit processed data, obtained by processing the raw data corresponding to a health object, to the electronic device 400. For example, the first server 410 may encrypt at least some of the genetic information and may transmit the encrypted data to the electronic device 400.

According to various exemplary embodiments, the first server 410 may request and obtain status information corresponding to at least one data item from the electronic device 400 or the external electronic device 420 and may request and obtain the genetic information from the second server 430. The first server 410 may transmit attribute information corresponding to the activity or state of the user to the electronic device 400 or the external electronic device 420 based on the genetic information and the status information.

According to various exemplary embodiments, the first server 410 may generate guide information based on the selected attribute information and may transmit the generated guide information to the electronic device 400 or the external electronic device 420. Upon receiving the guide information, the electronic device 400 or the external electronic device 420 may display graphical objects corresponding to the guide information on a display.

FIG. 5 illustrates the configuration of an electronic device according to various exemplary embodiments.

Referring to FIG. 5, the electronic device 400 (for example, the electronic device 101, 102, or 201) may include a processor 401 (for example, the processor 120 or 210), a display 402 (for example, the display 160 or 260), a sensor 403 (for example, the sensor module 180 or 240), a communication module 404 (for example, the communication interface 170 or the communication module 220), and a memory 405 (for example, the memory 130 or 230).

According to one exemplary embodiment, the processor 401 may select at least one data item among one or more health-related data items and may obtain status information corresponding to an activity or state of a user associated with the at least one selected item. The processor 401 may obtain genetic information on the user (for example, the user of the electronic device 400). The processor 401 may select attribute information on the activity or state of the user based on at least some of the obtained genetic information and may provide guide information associated with the activity or state of the user based on the selected attribute information.

According to one exemplary embodiment, the processor 401 may obtain status information (for example, information on the user's action (activity) associated with health (for example, information on food intake or water intake (for example, intake time/intake amount/degree), information on caffeine intake (for example, intake time/ intake amount/degree), information on exercise (for example, exercise time/exercise kind/calorie consumption), information on sleep (for example, sleeping time) or information on the user's body (for example, race/age/height/weight/waist size/heart rate/ oxygen saturation level/blood glucose level/blood pressure level/stress index)) associated with a data item corresponding to at least one health-related object (for example, a step count, walking, exercise, sleep, stress, caffeine, dietary management, water intake, heart rate, oxygen saturation, blood glucose, blood pressure, and stress).

According to various exemplary embodiments, the processor 401 may obtain the status information via the sensor 403, may obtain the status information by analyzing usage information (for example, payment details information, messages, application usage information, or SNS usage information) on the electronic device 400, or may obtain the status information by user input. The status information may be, for example, data including caffeine intake time/amount/degree or processed data representing an average recommended intake or above/below based on or medical or reliable data.

According to various exemplary embodiments, the processor 401 may obtain the status information from the external electronic device 420 connected via the communication module 404.

According to one exemplary embodiment, the processor 401 may transmit a request signal for requesting genetic information on the user to the first server 410 through the communication module 404 and may receive the genetic information from the first server 410. The genetic information may include gene information indicating various genetic traits which the user has.

According to various exemplary embodiments, the processor 401 may receive encrypted data (for example, encrypted genetic information) corresponding to at least some of the genetic information from the first server 410. The processor 401 may decrypt the encrypted data to obtain the genetic information and may store the obtained genetic information in an SE area of the memory 405.

According to one exemplary embodiment, the processor 401 may calculate the correlation between the obtained status information and the genetic information and may obtain priority information on the one or more data items based on the calculated correlation.

According to one exemplary embodiment, the processor 401 may compare genetic information corresponding to each of the one or more data items with standard sample data by category, such as race, disease, physical constitution, or reproducibility, may check a score depending on whether a specified condition is satisfied, and may determine the ranking of each data item by adding checked scores. For example, when the daily caffeine intake of the user with genes lacking an enzyme involved in caffeine degradation is greater than an average intake, the processor 401 may calculate a correlation therebetween to calculate a score for a data item corresponding to caffeine.

According to one exemplary embodiment, the processor 401 may compare analyzed genetic information with the standard sample data according to conditions specified by category in Table 1 below and may check a score according to the race of a user, scores according to disease and physical constitution, and scores according to reproducibility with respect to a disease or physical constitution.

**[Table 1]**

| Category | Condition | Score |
|---|---|---|
| Race | Korean | 9 |
| | Asian | 6 |
| | European and others | 3 |
| Disease | Both examples and controls are identical to 1000 samples or above | 3 |
| | Examples or controls are identical to 1000 samples or above | 2 |
| | Both examples and controls are identical to less than 1000 samples | 1 |
| Physical constitution | Identical to 8000 samples or above | 3 |
| | Identical to 4000 to no more than 8000 samples | 2 |
| | Identical to less than 4000 samples | 1 |
| Reproducibility | Reproduced in other racial groups | 3 |
| | Other valid data found in at least some of the same paper | 2 |
| | None | 1 |

For example, when the user is 'Korean' and has a gene lacking an enzyme involved in caffeine degradation associated with physical constitution, there are 5000 samples containing a gene identical to the gene, and the gene (or physical constitution) is reproducible in another racial group, the total score of the data item corresponding to caffeine may be 14 points by adding 9 points corresponding to race, 2 points corresponding to physical constitution, and 3 points corresponding to reproducibility.

According to various exemplary embodiments, the processor 401 may also calculate a score for one or more data items by applying a weight to at least some of the checked scores for the respective categories.

As described above, the processor 401 may calculate a scores for each data item based on Table 1 and may assign a ranking to each data item based on the calculated score.

According to one exemplary embodiment, the processor 401 may determine the position of the one or more data items to be displayed on the display 402 according to the obtained priority information and may display the one or more data items on the display 402 based on the determined position. For example, the processor 401 may display graphical objects corresponding to the one or more data items on the display 402. The data items may include a graphical object corresponding to information associated with the activity or state of the user as well as a graphical object corresponding to healthcare information.

According to one exemplary embodiment, the processor 401 may determine desired numerical information based on the genetic information, the status information, and rule information. The rule information may be set for the user corresponding to the genetic information and the status information (or data item). For example, the rule information may be information set to propose scalp management information in response to hair loss treatment for a user having a gene expressing a high hair loss index. In this case, the processor 401 may determine the scalp management information (for example, for recommending having scalp massages once in the morning and once in the afternoon or for recommending using hair loss prevention products) as desired numerical information.

According to one exemplary embodiment, the processor 401 may compare accumulated numerical information with the desired numerical information and may determine whether a comparison result satisfies a specified condition. When the comparison result satisfies the specified condition, the processor 401 may display a notification associated with the activity or state of the user on the display 402. When the desired numerical information is information limited to a specific numerical value, if the accumulated numerical information is close to or is greater than the desired numerical information, the processor 401 may display, on the display 402, a notification associated with the activity or state of the user (for example, information indicating that an accumulated value is close to a desired value or information indicating that an accumulated value is greater than or equal to a desired value) based on the genetic information, the state information, and the rule information. When the desired numerical information is information aimed at a specific numerical value, if the accumulated numerical information is less than or equal to the desired numerical information, the processor 401 may display, on the display 402, a notification associated with the activity or state of the user (for example, information indicating that an accumulated value is less than a desired value or information indicating that an accumulated value reaches a desired value) based on the genetic information, the state information, and the rule information.

For example, when desired numerical information for a user having a gene expressing a high hair loss index is one scalp massage each in the morning/afternoon (total two times), if it is determined that the user has had a scalp massage only once in the morning, the processor 401 may display a notification for recommending one afternoon scalp massage on the display 402. Further, when desired numerical information for a user having a gene expressing a high blood glucose increase rate is a meal of 2/3 bowls of multigrain rice, if it is determined that the user has had 2 bowls, the processor 401 may display a notification for warning of a risk by an increase in blood glucose level on the display 402.

According to various exemplary embodiments, the condition may be variously specified for each data item by genetic information, status information, and rule information.

According to various exemplary embodiments, when the activity or state of the user is changed, the processor 401 may update attribute information and may provide guide information associated with the activity or state of the user based on the updated attribute information.

For example, when the status information corresponding to the activity or state of the user is changed and thus it is determined that the user having a gene lacking an enzyme involved in caffeine degradation maintains the daily caffeine intake at an average intake or lower, the processor 401 may rerank the data item corresponding to caffeine and may rearrange the data items according to the reranking. The data item corresponding to caffeine may have a lower ranking than the previous ranking.

According to various exemplary embodiments, the processor 401 may transmit the genetic information or the status information to the first server 410 or the external electronic device 420 and may receive attribute information (for example, at least one of priority information, rule information, and desired numerical information) or guide information from the first server 410. The processor 401 may receive at least some of the status information from the external electronic device 420.

According to one exemplary embodiment, the display 402 may display an execution screen of a health-related application. The execution screen may include at least one item object, and each item object may include at least one graphical object associated with the activity or state of the user.

According to one exemplary embodiment, the display 402 may display the guide information. For example, the display 402 may display at least one graphical object corresponding to the guide information.

According to one exemplary embodiment, the sensor 403 may obtain at least some of the status information corresponding to the activity or state of the user. For example, the sensor 403 may include an accelerometer, a pedometer sensor, a gyro sensor, and a heart rate sensor.

According to one exemplary embodiment, the communication module 404 may communicate with the first server 410 and the external electronic device 420.

According to one exemplary embodiment, the memory 405 may store the genetic information.

According to one exemplary embodiment, the memory 405 may store information used to provide the guide information based on the genetic information and the status information. For example, the memory 405 may store rule information associated the genetic information on the user and the status information.

According to one exemplary embodiment, the memory 405 may include a general storage area and an SE area for storing information requiring security, and may store the genetic information in the SE area.

According to various exemplary embodiments, the memory 405 may store medical data, articles, research results, and the like.

According to various exemplary embodiments, the memory 405 may store information proposed by an expert, such as a doctor, a fitness instructor, or the like.

According to various exemplary embodiments, the memory 405 may store various pieces of information (for example, desired numerical information) on other users having genetic information similar to that of the user.

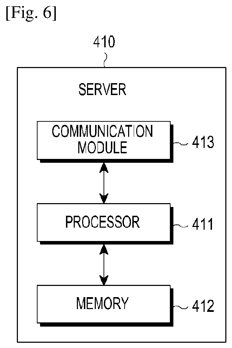

FIG. 6 illustrates the configuration of a first server according to various exemplary embodiments.

Referring to FIG. 6, the first server 410 may include a processor 411, a memory 412, and a communication module 413.

According to one exemplary embodiment, the processor 411 may obtain genetic information including a result of analyzing tissues collected from a user's tongue or hair and may store the obtained genetic information in an SE area of the memory 412.

When a request signal requesting the genetic information is received from an electronic device 400 or an external electronic device 420 via the communication module 413, the processor 411 may transmit a response signal including the genetic information to the electronic device 400 or the external electronic device 420 in response to the request. For example, the processor 411 may encrypt the genetic information and may transmit a response signal including the encrypted genetic information to the electronic device 400 or the external electronic device 420.

According to various exemplary embodiments, when a request signal requesting the genetic information is received from the electronic device 400 or the external electronic device 420, the processor 411 may request the genetic information from a second server 430. For example, the second server 430 may be a server that provides genetic information. When the genetic information is received from the second server 430, the processor 411 may transmit the received genetic information to the electronic device 400 or the external electronic device 420 or may encrypt and transmit the received genetic information to the electronic device 400 or the external electronic device 420.

According to various exemplary embodiments, when status information is received corresponding to one or more data items from the electronic device 400 or the external electronic device 420, the processor 411 may calculate priority information on the one or more data items based on the genetic information and the status information and may transmit the calculated priority information to the electronic device 400 or the external electronic device 420.

According to various exemplary embodiments, the processor 411 may transmit rule information associated with the genetic information and the status information to the electronic device 400 or the external electronic device 420 according to a request from the electronic device 400 or the external electronic device 420.

According to various exemplary embodiments, the processor 411 may calculate accumulated numerical information based on the status information and may transmit the calculated accumulated numerical information to the electronic device 400 or the external electronic device 420 according to a request from the electronic device 400 or the external electronic device 420.

According to various exemplary embodiments, the processor 411 may determine desired numerical information based on the genetic information, the status information, and the rule information and may transmit the determined desired numerical information to the electronic device 400 or the external electronic device 420 .

According to one exemplary embodiment, when updated status information corresponding to an activity or state of the user is received from the electronic device 400 or the external electronic device 420, the processor 411 may calculate (or determine) priority information, accumulated numerical information, and desired numerical information based on the genetic information and the updated status information and may transmit the calculated (or determined) priority information, accumulated numerical information, and desired numerical information to the electronic device 400 or the external electronic device 420.

According to various exemplary embodiments, the processor 411 may store various pieces of health-related information in the memory 412 and may transmit the health-related information according to a request from electronic device 400 or the external electronic device 420. For example, the various pieces of health-related information may include information on a disease or physical constitution, treatment or prevention information on a disease or physical constitution, information on proposed care in response to a disease or physical constitution (for example, dietary information, food information, exercise information, or experience information), and the like.

According to various exemplary embodiments, the processor 411 may determine the priority of at least one data item among the one or more data items based on the genetic information and may transmit information on the determined priority to the electronic device 400 or the external electronic device 420.

According to one exemplary embodiment, the memory 412 may store genetic information for at least one user.

According to one exemplary embodiment, the memory 412 may include a general storage area and an SE area, and may store the genetic information on the at least one user in the SE area.

According to various exemplary embodiments, the memory 412 may store the status information received from the electronic device 400 or the external electronic device 420 or may store the rule information, the priority information, the accumulated numeric information, and the desired numerical information .

According to various exemplary embodiments, the memory 412 may store medical data, articles, research results, and the like.

According to various exemplary embodiments, the memory 412 may store information proposed by an expert, such as a doctor, a fitness instructor, or the like.

According to various exemplary embodiments, the memory 412 may store various pieces information (for example, desired numerical information) on other users having genetic information similar to that of the user.

According to various exemplary embodiments, the memory 412 may store various pieces of health-related information.

According to one exemplary embodiment, the communication module 413 may communicate with the electronic device 400, the external electronic device 420, or the second server 430.

The electronic device 400 according to various exemplary embodiments may include: an output module (for example, the display 402); a memory 405 that stores one or more health-related data items, each of which includes a graphical object corresponding to an activity or state of a user; and a processor 401, wherein the processor 401 may: obtain status information corresponding to the activity or the state associated with at least one data item selected from the one or more data items; obtain genetic information on the user; select at least one piece of attribute information on the activity or state based on at least some of the genetic information; and provide guide information associated with the activity or the state through the output module based on the at least one piece of attribute information.

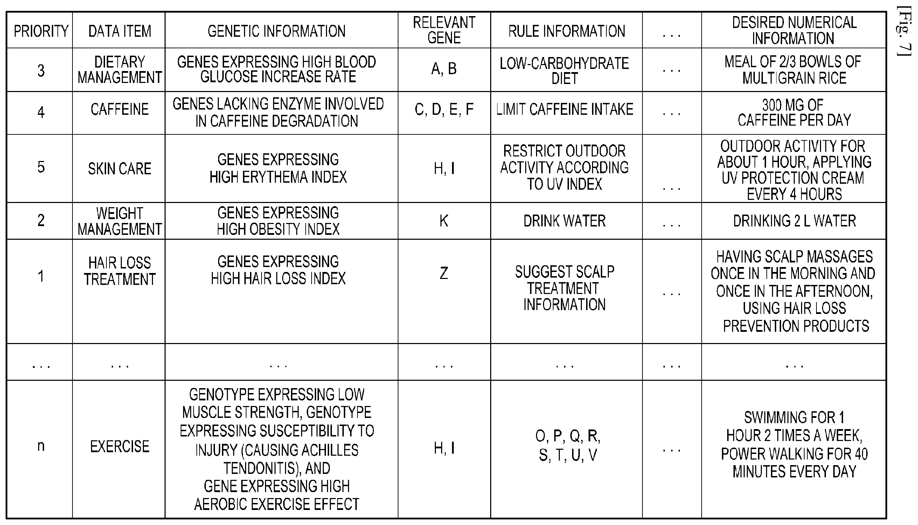

FIG. 7 illustrates an example of a table according to various exemplary embodiments.

According to FIG. 7, the table may include priority information corresponding to one or more data items, genetic information, information on a related gene, rule information, and desired numerical information. The table may be stored in the SE area of the memory 405 provided in the electronic device 400 or in the SE area of the memory 412 provided in the first server 410.

According to one exemplary embodiment, the electronic device 400 may obtain status information corresponding to an activity or state of a user corresponding to one or more data items, such as dietary management, caffeine, skin care, weight management, hair loss treatment, or exercise, and may obtain genetic information corresponding to one or more data items. The genetic information may include information on genes expressing a high blood glucose increase rate for dietary management, information on genes lacking an enzyme involved in caffeine degradation for caffeine, information on genes expressing a high erythema index for skin care, information on genes expressing a high obesity index for weight management, information on genes expressing a high hair loss index for hair loss treatment, and information on at least one gene (for example, a gene expressing low muscle strength, a gene expressing susceptibility to injury, and a gene expressing high aerobic exercise effect) for exercise.

According to one exemplary embodiment, the electronic device 400 may set rule information associated with the genetic information and the status information.

According to one exemplary embodiment, when it is determined that a user with a gene expressing a high blood glucose increase rate frequently eats food that leads to a larger increase in blood glucose level, the electronic device 400 may set a low-carbohydrate diet as rule information.

According to one exemplary embodiment, when it is determined that a user with a gene lacking an enzyme involved in caffeine degradation takes more than an average recommended amount of caffeine, the electronic device 400 may set information for limiting caffeine intake as rule information.

According to one exemplary embodiment, when it is determined that a user with a gene expressing a high erythema index frequently conducts outdoor activities, the electronic device 400 may set restriction of an outdoor activity according to UV index as rule information.

According to one exemplary embodiment, when it is determined that a user with a gene expressing a high obesity index drinks 0.5 L of water per day, the electronic device 400 may set water intake as rule information.

According to one exemplary embodiment, when it is determined that a user with a gene expressing a high hair loss index does not receive scalp treatment, the electronic device 400 may set a suggestion of scalp treatment information as rule information.

According to one exemplary embodiment, when it is determined that a user with a gene expressing low muscle strength, a gene expressing susceptibility to injury (for example, causing Achilles tendonitis), and a gene expressing high aerobic exercise effect does muscle workout, the electronic device 400 may set aerobic exercise, such as swimming or power walking, as rule information.

According to one exemplary embodiment, the electronic device 400 may store the set rule information in the memory 405 and may determine desired numerical information based on the genetic information, the status information, and the rule information.

For example, when rule information relating to dietary management is "a low-carbohydrate diet" and it is determined that a user frequently eats food that leads to a larger increase in blood glucose level, the electronic device 400 may determine information of "a meal of 2/3 bowls of multigrain rice" as desired numerical information.

When rule information relating to caffeine is "restricting caffeine intake" and the amount of coffee consumed by the user is determined to be greater than the average intake amount, the electronic device 400 may determine information of "200 mg of caffeine per day" as desired numerical information.

When rule information relating to skin care is "restricting an outdoor activity according to UV index" and it is determined that the user conducts an outdoor activity for 2 hours or longer, the electronic device 400 (for example, the processor 401) may determine information of "outdoor activity for about 1 hour and applying a UV protection cream every 4 hours" as desired numerical information.

When rule information relating to weight management is "drinking more water" and it is determined that the user drinks water less than the average intake, the electronic device 400 may determine information of "drinking 2 L water" as desired numerical information.

When rule information relating to hair loss treatment is "a suggestion of scalp treatment information" and it is determined that the user receives no scalp treatment, the electronic device 400 may set information of "having scalp massages once in the morning and once in the afternoon and using hair loss prevention products" as desired numerical information.

When rule information relating to exercise is "a suggestion of swimming and power walking" and it is determined that the user does muscle workout, the electronic device 400 may set information of "swim for 1 hour 2 times a week and power walk for 40 minutes every day" as desired numerical information.

According to various exemplary embodiments, the electronic device 400 may collect various pieces of health information (for example, information on treatment or prevention relating to a disease or physical constitution) associated with the genetic information or may be provided with the health information from the first server 410. The electronic device 400 may set rule information or may determine desired numerical information based on the various pieces of health information associated with the genetic information as well as the genetic information and the status information. Accordingly, the rule information or desired numerical information may include at least some of information for treatment or prevention relating to a disease or physical constitution associated with the genetic information.

According to various exemplary embodiments, the electronic device 400 may set desired numerical information for each data item based on the genetic information on the user. For example, when a user has a gene expressing a high obesity index, the electronic device 400 may set desired numerical information corresponding to individual data items associated with a gene expressing a high obesity index, such as dietary management, weight management, and exercise (for example, dietary management: 200 g protein intake per day, weight management: dinking 2 L water, and exercise: aerobic exercise for at least 30 minutes a day).

According to various exemplary embodiments, the electronic device 400 may determine the priorities of the data items using activity information on the user corresponding to the genetic information and an algorithm provided by a health-related application. The electronic device 400 may arrange each data item based on the determined priorities or may determine a data item for notification among the data items. For example, the electronic device 400 may arrange and display data items in the order of hair loss treatment, weight management, dietary management, caffeine, and exercise based on priority information illustrated in FIG. 7. Further, the electronic device 400 may determine data items related to hair loss treatment, weight management, and dietary management corresponding to the first rank, the second rank, and the third rank as data items for notification and may provide notification information for each of the data items.

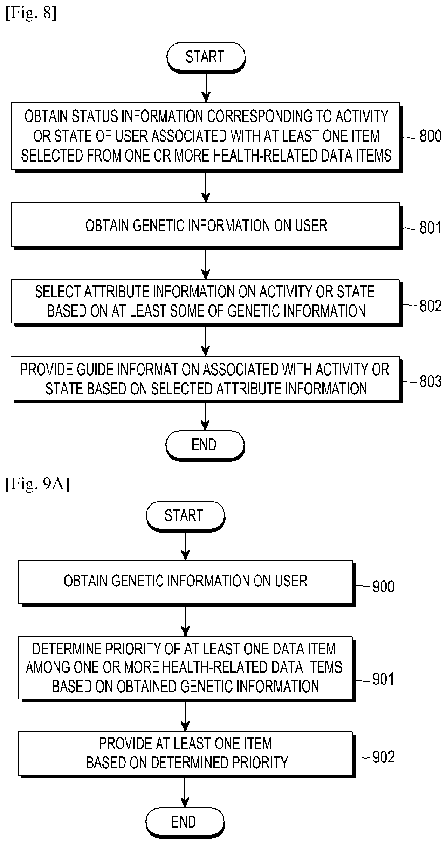

FIG. 8 is a flowchart illustrating that an electronic device provides guide information based on genetic information and status information according to various exemplary embodiments.

According to various exemplary embodiments, operations 800 to 803 may be performed by any one of the electronic device 101, 102, 104, 201, or 400, the server 106, 410 or 430, the processor 120, 210 401, or 411, and the program module 310.

Referring to FIG. 8, in operation 800, the electronic device 400 (for example, the processor 401) may obtain status information corresponding to an activity or state of a user associated with at least one item selected from one or more health-related data items.

For example, the electronic device 400 (for example, the processor 401) may select at least one data item among the one or more health-related data items based on user input. The data item may include a graphical object corresponding to the activity or state of the user.

The electronic device 400 (for example, the processor 401) may obtain the status information corresponding to the activity or state of the user associated with the at least one selected item, for example, using at least one sensor (for example, the sensor module 240) disposed in the electronic device 400.

In operation 801, the electronic device 400 (for example, the processor 401) may obtain at least some of genetic information on the user. For example, the electronic device 400 (for example, the processor 401) may check the genetic information stored in the memory 405 or may be provided with the genetic information from the first server 410.

In operation 802, the electronic device 400 (for example, the processor 401) may select attribute information on the activity or state based on at least some of the genetic information. The attribute information may include priority information, accumulated numerical information, and desired numerical information.

In operation 803, the electronic device 400 (for example, the processor 401) may provide guide information associated with the activity or state based on the selected attribute information. The guide information may include at least one data item provided based on the priority information or may include a notifications associated with the activity or state.

FIG. 9A is a flowchart illustrating that an electronic device provides guide information based on genetic information and status information according to various exemplary embodiments.

According to various exemplary embodiments, operations 900 to 902 may be performed by any one of the electronic device 101, 102, 104, 201, or 400, the server 106, 410, or430, the processor 120, 210 401, or 411, and the program module 310.

Referring to FIG. 9A, in operation 900, the electronic device 400 (for example, the processor 401) may obtain genetic information on a user.

In operation 901, the electronic device 400 (for example, the processor 401) may determine the priority of at least one data item among one or more health-related data items based on the obtained genetic information. The priority may be determined based on a risk level (or information) relating to a disease or physical constitution corresponding to at least one data item.

In operation 902, the electronic device 400 (for example, the processor 401) may provide at least one item based on the determined priority.

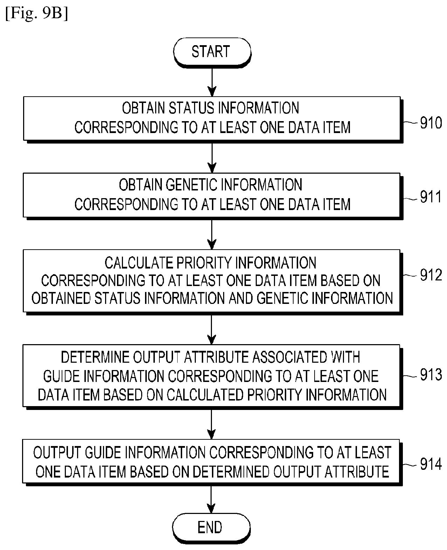

FIG. 9B is a flowchart illustrating that an electronic device provides guide information based on genetic information and status information according to various exemplary embodiments.

According to various exemplary embodiments, operations 910 to 914 may be performed by any one of the electronic device 101, 102, 104, 201, or 400, the server 106 410, or430, the processor 120, 210 401, or 411, and the program module 310.

Referring to FIG. 9B, in operation 910, the electronic device 400 (for example, the processor 401) may obtain status information corresponding to at least one data item.

In operation 911, the electronic device 400 (for example, the processor 401) may obtain genetic information corresponding to the at least one data item.

In operation 912, the electronic device 400 (for example, the processor 401) may calculate priority information corresponding to the at least one data item based on the obtained status information and genetic information.

In operation 913, the electronic device 400 (for example, the processor 401) may determine an output attribute associated with guide information corresponding to the at least one item based on the calculated priority information.

In operation 914, the electronic device 400 (for example, the processor 401) may output the guide information corresponding to the at least one data item based on the determined output attribute. According to one exemplary embodiment, the electronic device 400 (for example, the processor 401) may place the at least one data item in order from the highest ranking to the lowest ranking based on the determined output attribute. According to various exemplary embodiments, the electronic device 400 (for example, the processor 401) may provide a notification associated with an activity or state based on the determined output attribute.

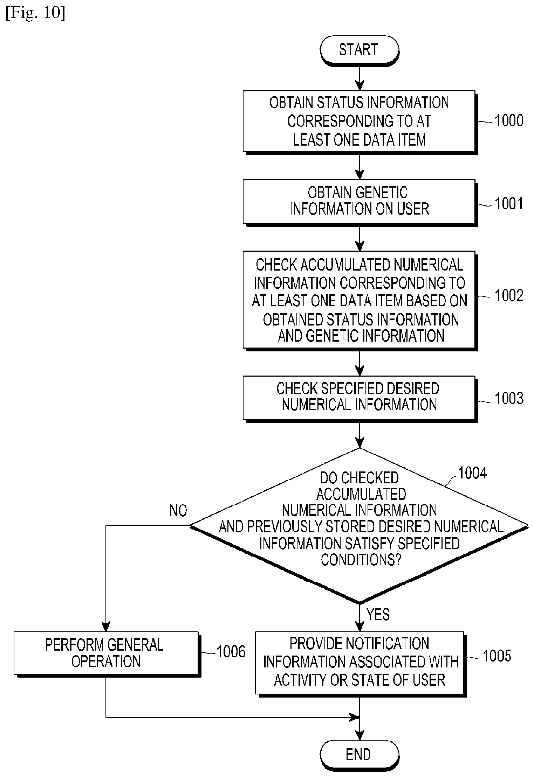

FIG. 10 is a flowchart illustrating that an electronic device provides guide information based on genetic information and status information according to various exemplary embodiments.

According to various exemplary embodiments, operations 1000 to 1006 may be performed by any one of the electronic device 101, 102, 104, 201, or 400, the server 106, 410 or 430, the processor 120, 210 401, or 411, and the program module 310.

Referring to FIG. 10, in operation 1000, the electronic device 400 (for example, the processor 401) may obtain status information corresponding to at least one data item.

In operation 1001, the electronic device 400 (for example, the processor 401) may obtain genetic information on a user. For example, the electronic device 400 (for example, the processor 401) may be provided with the genetic information from the first server 410.

In operation 1002, the electronic device 400 (for example, the processor 401) may check accumulated numerical information corresponding to the at least one data item based on the obtained status information and genetic information. For example, the accumulated numerical information may be "daily caffeine intake: 300 mg."

In operation 1003, the electronic device 400 (for example, the processor 401) may check specified desired numerical information. For example, the desired numerical information may be "caffeine intake limit: 200 mg or less (or coffee intake limit: 3 cups)."

In operation 1004, the electronic device 400 (for example, the processor 401) may determine whether the checked accumulated numerical information and previously stored desired numerical information satisfy specified conditions. When the specified conditions are satisfied, operation 1005 is performed. When the specified conditions are not satisfied, the electronic device 400 may perform a general operation in operation 1006.

In operation 1005, the electronic device 400 (for example, the processor 401) may provide notification information associated with an activity or state of the user. For example, the electronic device 400 (for example, the processor 401) may compare the daily caffeine intake with a caffeine intake limit to determine whether the daily caffeine intake exceeds the caffeine intake limit. When the daily caffeine intake exceeds the caffeine intake limit, the electronic device 400 (for example, the processor 401) may provide notification information indicating that the daily caffeine intake exceeds the caffeine intake limit. For example, the notification information may further include information for reporting (or warning of) the risk of excessive consumption of caffeine in association with to the genetic information on the user.

A method of an electronic device 400 including an output module (for example, the display 402), a memory 405 that stores one or more health-related data items, and a processor 401 according to various exemplary embodiments may include: obtaining status information corresponding to an activity or state of a user associated with at least one data item selected from the one or more data items; obtaining genetic information on the user; selecting at least one piece of attribute information on the activity or state, using the processor 401, at least based on the genetic information; and providing guide information associated with the activity or the state through the output module based on the at least one piece of attribute information.

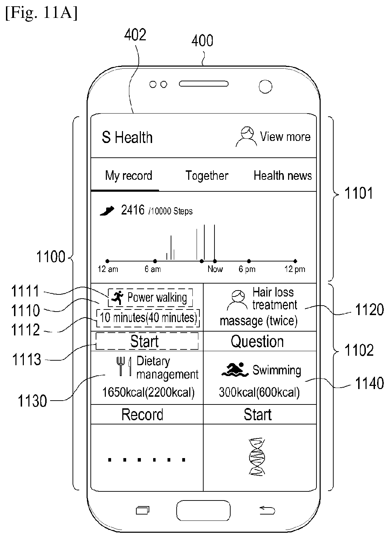

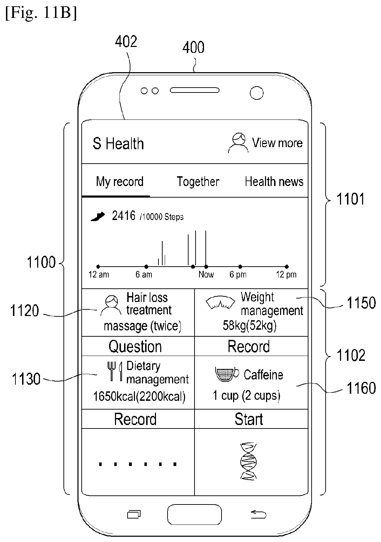

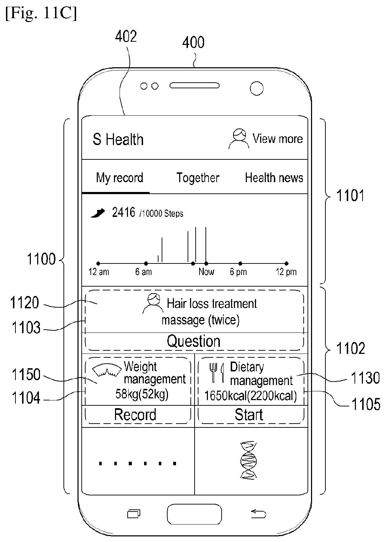

FIGS. 11A, 11B, and 11C illustrate examples of a user interface associated with a healthcare service according to various exemplary embodiments.

Referring to FIG. 11A, the electronic device 400 may display, on the display 402, an execution screen 1100 of a health-related application in response to input for running the health-related application. The execution screen 1100 may be a main screen of the health-related application.

The execution screen 1100 may include a first area 1101 for displaying daily step count information (for example, a graph) and a second area 1102 for displaying one or more data items, such as data items 1110, 1120, 1130, and 1140. The data items may include a graphical object (for example, an image or a text) representing a health-related health factor (for example, power walking, hair loss treatment, dietary management, or swimming), a graphical object (for example, a text) representing accumulated numerical information and desired numerical information corresponding to a health factor, and a graphical object (for example, an icon including a text and an image) corresponding to a function (for example, Detailed Information Setting) associated with a data item. For example, a data item 1110 relating to power walking may include an image and text 1111 representing "power walking" corresponding to a health factor, a text 1112 representing accumulated numerical information (for example, 10 minutes) and desired numerical information (for example, 40 minutes) on power walking, and an icon 1113 corresponding to a function (for example, Detailed Information Setting, such as Quick Access (Start/Stop), View Detailed Information, Modify Record, or Question to Expert) associated with the data item relating to power walking.

According to one exemplary embodiment, the electronic device 400 may obtain status information corresponding to the one or more data items, may obtain genetic information on a user, and may obtain priority information on the one or more data items based on the obtained genetic information and status information. The electronic device 400 may determine an output attribute (for example, an output position) of each data item based on the obtained priority information.

For example, when priorities of at least one item are in the order of "power walking, hair loss treatment, dietary management, and swimming," the electronic device 400 may display the data item 1110 relating to power walking at a first position (for example, the top left of the second area 1102), may display a data item 1120 relating to hair loss treatment at a second position (for example, the top right of the second area 1102), may display a data item 1130 relating to dietary management at a third position (for example, under the first position), and may display a data item 1140 relating to swimming at a fourth position (for example, under the second position) in order to show a data item with a higher rank first.

According to various exemplary embodiments, the electronic device 400 may display the data items in the order of hair loss treatment, weight management, dietary management, and caffeine based on the priority information determined in FIG. 7.

Referring to FIG. 11B, when the status information corresponding to an activity or state of the user is changed by a change in the activity or state of the user, the electronic device 400 may update the priority information based on the changed status information. The electronic device 400 may determine an output attribute (for example, an output position) of each data item based on the updated priority information.

For example, when accumulated values associated with power walking and swimming for a specified period exceed desired values and accumulated values associated with hair loss treatment, weight management, dietary management, and caffeine do not reach desired values, the data items relating to power walking and swimming falls in rankings and the data items relating to hair loss treatment, weight management, dietary management, and caffeine rise in rankings.

When priorities of at least one item are in the order of "hair loss treatment, weight management, dietary management, and caffeine," the electronic device 400 may display the data item 1120 relating to hair loss treatment at the first position (for example, the top left of the second area 1102), may display a data item 1150 relating to weight management at the second position (for example, the top right of the second area 1102), may display the data item 1130 relating to dietary management at the third position (for example, under the first position), and may display a data item 1160 relating to caffeine at the fourth position (for example, under the second position) in order to show a data item with a higher rank first.

Referring to FIG. 11C, the electronic device 400 may display at least one graphical object included in each data item with a different size according to importance (or priority).

For example, when priorities of at least one item are in the order "hair loss treatment, weight management, and dietary management," the electronic device 400 may display a data item 1120 relating to hair loss treatment, which is determined to have the highest importance, with a first size in an area 1103 (for example, the upper part of the second area 1102) corresponding to the first position and the second position. The first size may be the size of the area 1103 corresponding to the first position and the second position.

The electronic device 400 may display the data item 1150 relating to weight management, which has lower importance than hair loss treatment, with a second size smaller than the first size in an area 1104 corresponding to the third position, and may display the data item 1130 relating to dietary management, which has lower importance than hair loss treatment and weight management, with a third size in an area 1105 corresponding to the fourth position. The third size may be equal to or smaller than the second size.

According to various exemplary embodiments, at least one graphical object included in each data item may be set using a different color according to risk level. For example, a data item determined to have a high risk level may be represented by a red graphical object, while a data item determined to have a low risk level may be represented by a blue graphical object.

According to various exemplary embodiments, one or more data items determined to have a high risk level may be represented by a graphical object in a dark or light color according to risk level. For example, a data item having the highest risk level may be represented by a graphical object in a dark color, while a data item having the lowest risk level may be represented by a graphical object in a light color. Accordingly, the user may be notified of the risk level of each data item.

According to various exemplary embodiments, when there are multiple graphical objects corresponding to a genetic factor having a high risk level, the graphical objects may be arranged in order of direct or fatal connection to health. For example, priorities may be set in order from a disease group (for example, diabetes) to a physical constitution group (for example, caffeine).

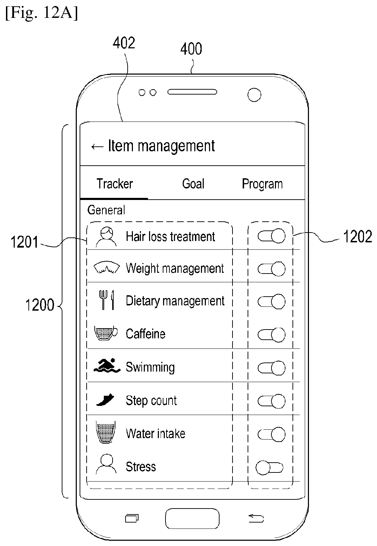

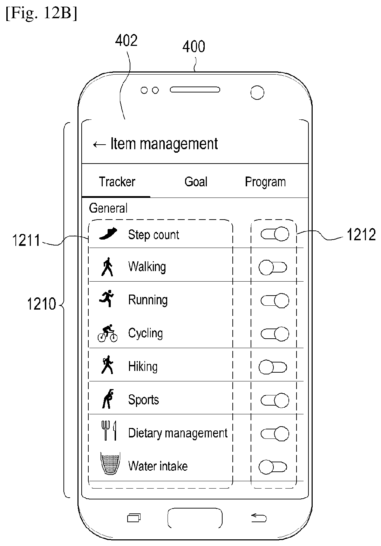

FIGS. 12A and 12B illustrate examples of one or more data items according to various exemplary embodiments.

Referring to FIG. 12A, the electronic device 400 may display, on the display 402, a user interface 1200 for activating at least one data item to be provided for a user among one or more data items. The user interface 1200 may include may include a graphical object 1201 (for example, an image or a text) representing a health factor (for example, hair loss treatment, weight management, dietary management, caffeine, swimming, a step count, water intake, or stress) corresponding to the one or more data items and graphical objects 1202 (for example, a button) for activating or deactivating one or more data items. When the button is moved to the right, a corresponding graphical object may be activated. When the button is moved to the left, the corresponding graphical object may be deactivated. At least one activated data item may be displayed in the second area 1102 of the execution screen 1100 of the health-related application as illustrated in FIG. 11A.

According to one exemplary embodiment, the electronic device 400 may calculate priority information on the one or more data items based on status information and genetic information on the user and may select at least one data item to be provided for the user based on the calculated priority information. The electronic device 400 may arrange a graphical object corresponding to the at least one selected data item in order of priority and may activate the at least selected one data item. For example, when priorities of the at least one selected data item are in the order of "hair loss treatment, weight management, dietary management, caffeine, swimming, a step count, water intake, and stress," the electronic device 400 may arrange and display the graphical objects 1201 corresponding to the at least one data item according to the priorities and may activate the at least one data item. As such, by activating or deactivating one or more data items based on the status information and genetic information on the user, data item management may automatically be conducted without the user implementing any separate operation.

When the status information is changed as the activity or state of the user is changed, the electronic device 400 may update the priority information based on the changed status information and the genetic information and may select data items to be provided for the user based on the updated priority information. The electronic device 400 may arrange the graphical objects 1201 corresponding to the selected data items in order of priority and may activate the selected data items. As such, as the priority information is updated, one or more data items provided for the user may also be updated.

Referring to FIG. 12B, the electronic device 400 may display, on the display 402, a user interface 1210 for activating at least one data item to be provided for the user among one or more data items. The user interface 1210 may include may include a graphical object 1211 (for example, an image or a text) representing a health factor (for example, a step count, walking, running, cycling, hiking, sports, dietary management, or water intake) corresponding to the one or more data items and graphical objects 1212 (for example, a button) for activating or deactivating one or more data items.

According to various exemplary embodiments, the electronic device 400 may calculate priority information on the one or more data items based on status information and genetic information on the user and may select at least one data item to be provided for the user based on the calculated priority information. The electronic device 400 may activate the at least one selected data item. For example, when the at least one selected data item includes a step count, running, cycling, sports, and dietary management, the electronic device 400 may activate the at least one selected data item and may provide the at least one activated data item based on the priority information. The at least one activated data item may be arranged and displayed in order of priority on the execution screen of the health application.

According to various exemplary embodiments, the electronic device 400 may activate at least one selected data item based on the genetic information and status information on the user and may deactivate at least one data item that is not selected.

According to various exemplary embodiments, the electronic device 400 may activate at least some data items that are determined to have a higher priority among the at least one selected data item and may deactivate at least some data items that are determined to have a lower priority.

According to various exemplary embodiments, FIGS. 12A and 12B are not limited to the one or more data items illustrated in the drawings, but a new data item may be added upon request from the user or a new data item including some data items may be added. Each of these data items may be added, changed, combined, or deleted without restraint.

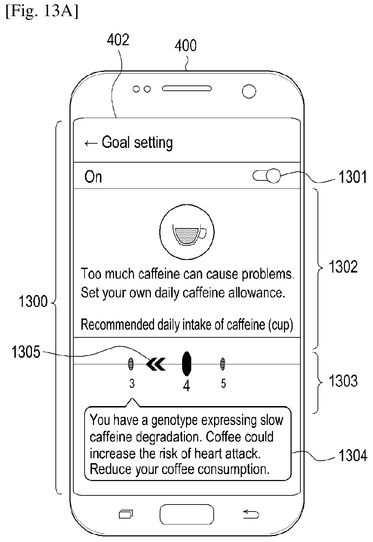

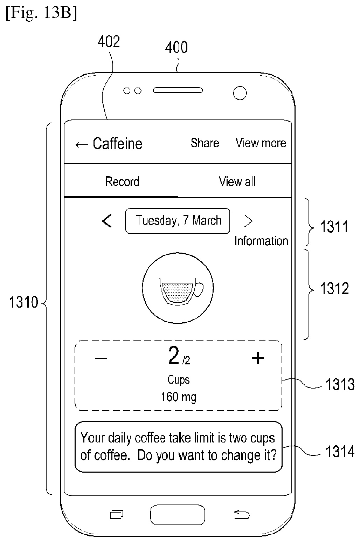

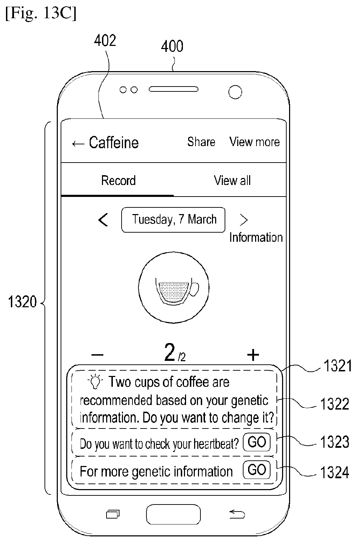

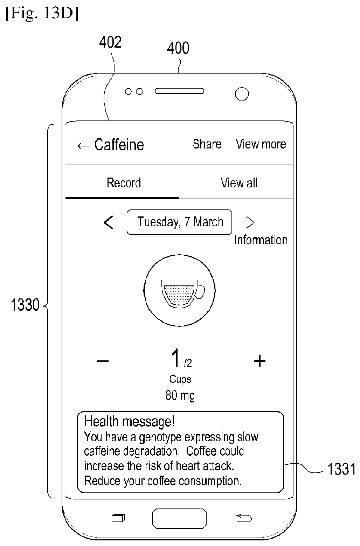

FIGS. 13A, 13B, 13C, and 13D illustrate examples of guide information according to various exemplary embodiments.

Referring to FIG. 13A, the electronic device 400 may display, on the display 402, a user interface 1300 for setting a desired value of a particular data item. The user interface 1300 may include a graphical object 1301 (for example, a button) corresponding to a function for activating the particular data item, a graphical object 1302 (for example, an image or a text) associated with the particular data item, and a graphical object 1303 corresponding to a function for setting the desired value corresponding to the particular data item. The electronic device 400 may display guide information 1304 corresponding to the data item based on genetic information and status information according to input for setting the desired value.

For example, when the recommended daily intake of caffeine for the user, which is identified based on the genetic information on the user, is three cups of coffee and input for setting a limit value to four cups of coffee is received, the electronic device 400 may display the guide information 1304 including notification information for reporting that the user has a gene expressing slow caffeine degradation, for indicating the risk of excessive consumption of caffeine, and for advising (or suggesting) restricting caffeine intake. The guide information 1304 may include a graphical object, for example, a text saying "You have a genotype expressing slow caffeine degradation. Coffee could increase the risk of heart attack. Reduce your coffee consumption."

The electronic device 400 may further display a separate graphical object 1305 (for example, an arrow icon) for suggesting changing the limit value from four cups of coffee to three cups of coffee.

Referring to FIG. 13B, the electronic device 400 may display, on the display 402, a user interface 1310 for entering (or setting) accumulated numerical information on the particular data item. The user interface 1310 may include a graphical object 1311 for selecting a specific date, a graphical object 1312 (for example, an image) associated with the particular data item, and a graphical object 1313 corresponding to a function for entering (or setting) the accumulated numerical information on the particular data item. The electronic device 400 may display guide information 1314 corresponding to the data item based on the genetic information and the status information when the accumulated numerical information and desired numerical information satisfy specified conditions.

For example, when the user has a gene expressing easy caffeine degradation, a caffeine intake limit is "two cups of coffee," and an accumulated caffeine intake is "two cups of coffee," the electronic device 400 may determine whether the current caffeine intake affects the user's health based on the genetic information and status information on the user. When it is determined that the current caffeine intake does not affect the user's health, the electronic device 400 may display the guide information 1314 including notification information for suggesting resetting the desired numerical information. The guide information 1314 may include a graphical object, for example, a text saying "Your daily coffee take limit is two cups of coffee. Do you want to change it"

Referring to FIG. 13C, the electronic device 400 may display a user interface 1320 for entering (or setting) accumulated numerical information on a data item relating to caffeine. The user interface 1320 may include guide information 1321 on caffeine.

For example, when the user has a gene lacking an enzyme involved in caffeine degradation, a caffeine intake limit is "two cups of coffee," and an accumulated caffeine intake is "two cups of coffee," the electronic device 400 may display guide information 1321 including a graphical object 1322 corresponding to recommendation information on a caffeine intake (or a coffee intake) proposed to the user based on the genetic information and the status information, a graphical object 1323 corresponding to a function for measuring heart rate, and a graphical object 1324 corresponding to a function for providing the genetic information on the user.

Referring to FIG. 13D, the electronic device 400 may display a user interface 1330 for entering the accumulated numerical information on the data item relating to caffeine. The user interface 1330 may include guide information 1331 on caffeine.

For example, when the user has a gene lacking an enzyme involved in caffeine degradation, the electronic device 400 may display, based on the genetic information, guide information 1331 (for example, a message for health) including a graphical object, for example, a text saying "You have a genotype expressing slow caffeine degradation. Coffee could increase the risk of heart attack. Reduce your coffee consumption."

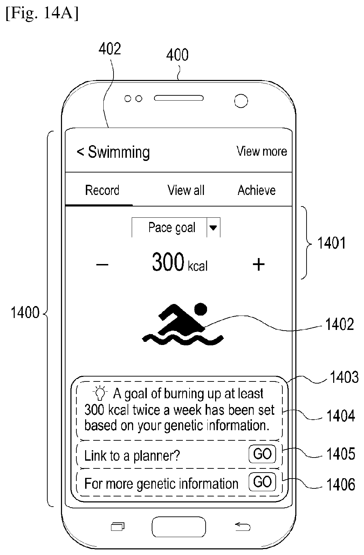

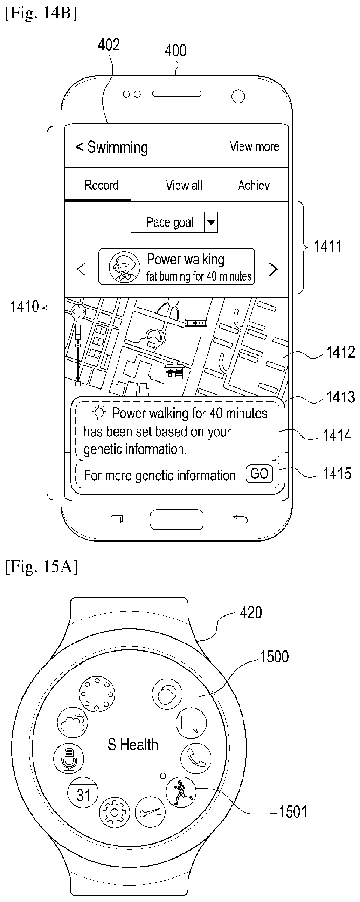

FIGS. 14A and 14B illustrate examples of guide information according to various exemplary embodiments.

Referring to FIG. 14A, the electronic device 400 may display, on the display 402, a user interface 1400 for setting desired numerical information corresponding to a particular data item according to input. The user interface 1400 may include a graphical object 1401 associated with the desired numerical information corresponding to the particular data item, a graphical object 1402 (for example, an image) associated with the particular data item, and guide information 1403 associated with the desired numerical information corresponding to the particular data item.

For example, when input for setting desired numerical information on swimming is received, the electronic device 400 may set desired numerical information on swimming based on genetic information and status information on the user and may display a graphical object 1401 representing the set desired numerical information and guide information 1403 on the set desired numerical information. When the set desired numerical information is "two times a week and 300 kcal or more," the guide information 1403 may include notification information 1404 for reporting the set desired numerical information, a graphical object 1405 corresponding to a function for executing a relevant application, and a graphical object 1406 corresponding to a function for providing the genetic information. The notification information 1404 may include a text saying "A goal of burning up at least 300 kcal twice a week has been set based on your genetic information." The graphical object 1405 may include an icon corresponding to a function for executing a specific application (for example, a planner) together with a text saying "Link to a planner?" The graphical object 1406 may include an icon for providing the genetic information on the user along with a text saying "For more genetic information."

Referring to FIG. 14B, when input for setting a desired value for a data item relating to running is received, the electronic device 400 may display, on the display 402, a user interface 1410 for setting desired numerical information on running. The user interface 1410 may include a graphical object 1411 associated with desired numerical information on running, a graphical object 1412 corresponding to map information indicating the traveling path of the user, and guide information 1413 on the desired numerical information.

For example, when input for setting desired numerical information on running is received, the electronic device 400 may set desired numerical information on running based on the genetic information and status information on the user and may display a graphical object 1411 representing the set desired numerical information and guide information 1413 on the set desired numerical information. When the set desired value information is "power walking for 40 minutes," the guide information 1413 may include notification information 1414 for reporting the set desired numerical information and a graphical object 1415 corresponding to a function for providing the genetic information. The notification information 1414 may include a text saying "Power walking for 40 minutes has been set based on your genetic information." The graphical object 1415 may include an icon for providing the genetic information on the user along with a text saying " For more genetic information."

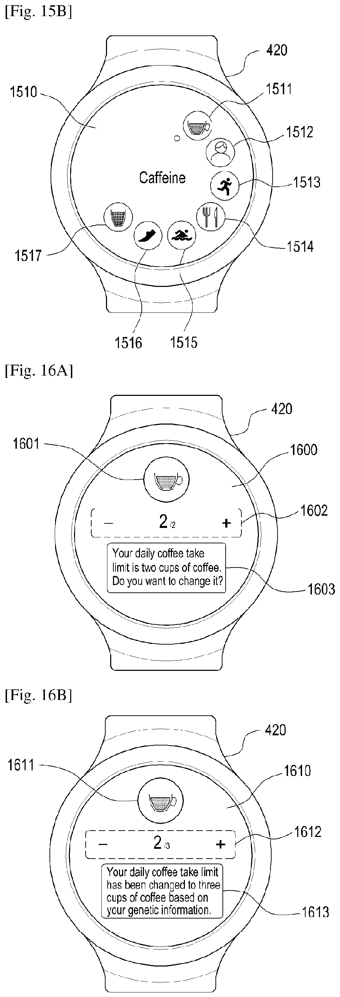

FIGS. 15A and 15B illustrate examples of guide information according to various exemplary embodiments. According to various exemplary embodiments, the external electronic device 420 may include a wearable device, such as a watch.

Referring to FIG. 15A, the external electronic device 420 may provide a user interface 1500 including a graphical object 1501 associated with a health-related application (for example, S Health). The graphical object 1501 may correspond to a function for executing the health-related application.

According to one exemplary embodiment, when input for executing the health-related application is received, the external electronic device 420 may provide a user interface 1510 corresponding to an execution screen, as illustrated in FIG. 15B.

Referring to FIG. 15B, the user interface 1510 may include graphical objects 1511, 1512, 1513, 1514, 1515, 1516, and 1517 corresponding to at least one data item among one or more health-related data items.

According to one exemplary embodiment, the external electronic device 420 may obtain status information corresponding to an activity or state of a user and may obtain genetic information on the user. For example, the external electronic device 420 may check the genetic information stored in the memory provided in the external electronic device 420 or may request the genetic information from the electronic device 400 or the first server 410 to receive the genetic information from the electronic device 400 or the first server 410.

The external electronic device 420 may select at least one data item from the one or more data items based on the obtained status information and genetic information and may calculate priority information on the at least one selected data item. The external electronic device 420 may determine an output attribute for each data item based on the calculated priority information, and may arrange and display at least one data item based on the determined output attribute.

For example, when the priority information includes the priorities of caffeine, hair loss treatment, power walking, dietary management, swimming, a step count, and water intake in order, as illustrated in FIG. 15B, the external electronic device 420 may clockwise arrange and display a graphical object 1511 corresponding to a data item relating to caffeine, a graphical object 1512 corresponding to a data item relating to hair loss treatment, a graphical object 1513 corresponding to a data item relating to power walking, a graphical object 1514 corresponding to a data item relating to dietary management, a graphical object 1515 corresponding to a data item relating to swimming, a graphical object 1516 corresponding to a data item relating to a step count, and a graphical object 1517 corresponding to a data item relating to water intake.

FIGS. 16A and 16B illustrate examples of guide information according to various exemplary embodiments. According to various exemplary embodiments, the external electronic device 420 may include a wearable device, such as a watch.

Referring to FIG. 16A, the external electronic device 420 may display a user interface 1600 for entering (or setting) accumulated numerical information corresponding to a data item relating to caffeine. The user interface 1600 may include a graphical object 1601 (for example, an image) relating to caffeine and a graphical object 1602 corresponding to a function for entering (or setting) accumulated numerical information corresponding to the data item relating to caffeine. The external electronic device 420 may compare the accumulated numerical information and desired numerical information. When a specified condition is satisfied, the external electronic device 420 may display guide information 1603 corresponding to the data item relating to caffeine based on genetic information and status information.

For example, when the user has a gene expressing easy caffeine degradation, the desired numerical information is "two cups of coffee," and the accumulated numerical information is "two cups of coffee," the external electronic device 420 may determine whether the current caffeine intake affects the user's health based on the genetic information and status information on the user. When it is determined that the current caffeine intake does not affect the user's health, the external electronic device 420 may display the guide information 1603 including notification information for suggesting resetting the desired numerical information. The guide information 1603 may include a graphical object, for example, a text saying "Your daily coffee take limit is two cups of coffee. Do you want to change it?"

Referring to FIG. 16B, the external electronic device 420 may display a user interface 1610 for entering (or setting) accumulated numerical information corresponding to the data item relating to caffeine. The user interface 1610 may include a graphical object 1611 (for example, an image) relating to caffeine, a graphical object 1612 corresponding to a function for entering (or setting) accumulated numerical information corresponding to the data item relating to caffeine, and guide information 1613 on caffeine.

For example, when desired numerical information on caffeine is "two cups of coffee" and the user has a gene expressing fast metabolism, the external electronic device 420 may reset the desired numerical information to "three cups of coffee" based on the user's genetic information and status information. The external electronic device 420 may display the guide information 1613 including notification information for reporting the reset desired numerical information. The notification information may include a graphical object, for example, a text saying "Your daily coffee take limit has been changed to three cups of coffee based on your genetic information."

According to various exemplary embodiments, the user interfaces of FIGS. 11A, 11B, 12A, 12B, 13A, 13B, 13C, 13D, 14A, 14B, 15A, 15B, 16A, and 16B are not limited to the above descriptions. At least some of the user interfaces shown in the drawings may be variously embodied, and user interfaces may be added, changed, deleted, or combined without restraint.

According to various exemplary embodiments of the present disclosure, more accurate and useful healthcare services may be provided for a user based on genetic information on the user and status information corresponding to the activity or state of the user.

As used herein, the term "module" may refer to a software unit, a hardware unit, or a firmware unit and may be used interchangeably with, for example, a logic, a logical block, a component, or a circuit. A module may be an integrated component, a minimum unit performing one or more functions, or a part thereof. The module may be implemented mechanically or electronically. For example, the module may include Application-Specific Integrated Circuit (ASIC) chips, Field-Programmable Gate Arrays (FPGAs), or Programmable Logic Devices that perform operations and have already been known or will be developed in the future. At least part of the devices (for example, modules or functions thereof) or methods (for example, operations) according to various exemplary embodiments may be implemented as instructions stored in a computer-readable recording medium (for example, the memory 130) in the form of a programming module. When the instructions are executed by a processor (for example, the processor 120), the processor may perform functions corresponding to the instructions. The computer-readable storage medium may include a hard disk, a floppy disk, a magnetic medium (for example, a magnetic tape), an optical medium (for example, a CD-ROM and a DVD), a magneto-optical medium (for example, a floptical disk), an internal memory, or the like. The instructions may include a code created by a compiler or a code executable by an interpreter.

A computer-readable recording medium according to various exemplary embodiments may record a program to implement, using a processor 401, a method in an electronic device 400 including a display 402, a memory 405 that stores one or more health-related data items, each of which includes a graphical object corresponding to an activity or state of a user, and the processor 401, wherein the method may include: obtaining genetic information on the user; determining a priority of at least one data item among the one or more data items at least based on the genetic information; and displaying the at least one data item through the display 402 according to the priority.

The programming module according to the present disclosure may include one or more of the aforementioned elements or may further include other additional elements, or some of the aforementioned elements may be omitted. Operations performed by a module, a programming module, or other elements according to various embodiments may be executed sequentially, in parallel, repeatedly, or in a heuristic manner. At least some operations may be executed according to another sequence, may be omitted, or may further include other operations.

Although the present disclosure has been described with an exemplary embodiment, various changes and modifications may be suggested to one skilled in the art. It is intended that the present disclosure encompass such changes and modifications as fall within the scope of the appended claims.

## Claims

1. An electronic device (101, 102, 104, 201, 400, 420) comprising:
an output module;
a memory (130, 405) configured to store health-related data items, wherein each of the health-related data items includes a graphical object corresponding to an activity or a state of a user; and
a processor (120, 210, 401, 411) configured to:
obtain status information corresponding to the activity or the state associated with at least one data item selected from the health-related data items,
obtain genetic information on the user,
calculate priority information on the health-related data items by comparing genetic information corresponding to each of the health-related data items with standard sample data by category, checking a score of each of the health-related data items depending on whether the comparison result meets a specified condition, and determining a ranking of each of the health-related data items by adding the checked scores,
select at least part of attribute information on the activity or the state based on at least part of the genetic information, wherein the attribute information comprises the priority information on the health-related data items calculated based on the genetic information and the status information,
obtain guide information associated with the health-related data items, and
provide the guide information through the output module based on at least part of the priority information, wherein the guide information is arranged based on the priority information,
wherein the category includes race, disease, physical constitution, or reproducibility with respect to the race, the disease or the physical constitution.

2. The electronic device of claim 1, further comprising a communication module (220),
wherein the processor is configured to receive at least part of the genetic information from an external electronic device through the communication module.

3. The electronic device of claim 2, wherein the processor is configured to:
receive encrypted data corresponding to at least part of the genetic information as at least part of an operation of receiving the at least part of the genetic information; and
obtain the genetic information by decrypting the encrypted data.

4. The electronic device of claim 1, wherein the processor is configured to store the obtained genetic information in the memory.

5. The electronic device of claim 4, wherein the memory comprises a Security Enhancement (SE) area,
wherein the processor is configured to store the obtained genetic information in the SE area.

6. The electronic device of claim 1, further comprising a sensor,
wherein the processor is configured to obtain at least part of the status information based on at least part of data detected through the sensor.

7. The electronic device of claim 1, wherein the processor is configured to update accumulated numerical information based on at least part of the status information.

8. The electronic device of claim 7,
wherein the processor is configured to:
compare the accumulated numerical information and desired numerical information; and
provide a notification associated with the activity or the state via the output module when a comparison result satisfies a specified condition.

9. A computer-implemented method, the method comprising:
obtaining status information corresponding to an activity or a state of a user associated with at least one data item selected from health-related data items;
obtaining genetic information on the user;
calculating priority information on the health-related data items by comparing genetic information corresponding to each of the health-related data items with standard sample data by category, checking a score of each of the health-related data items depending on whether the comparison result meets a specified condition, and determining a ranking of each of the health-related data items by adding the checked scores;
selecting at least part of attribute information on the activity or the state based on the genetic information, wherein the attribute information comprises the priority information on the health-related data items calculated based on the genetic information and the status information;
obtaining guide information associated with the health-related data items; and
providing the guide information through an output module based on at least part of the priority information, wherein the guide information is arranged based on the priority information,
wherein the category includes race, disease, physical constitution, or reproducibility with respect to the race, the disease or the physical constitution.

10. The method of claim 9, wherein the method further comprises updating accumulated numerical information based on at least part of the status information.

11. The method of claim 10, wherein the method further comprises:
comparing the accumulated numerical information and desired numerical information; and
providing a notification associated with the activity or the state through the output module when a comparison result satisfies a specified condition.

12. A non-transitory computer-readable medium, embodying a computer program, the computer program comprising code that when executed by at least one processor of an electronic device causes the at least one processor to:
obtain status information corresponding to an activity or a state associated with at least one data item selected from health-related data items,
obtain genetic information on the user,
calculate priority information on the health-related data items by comparing genetic information corresponding to each of the health-related data items with standard sample data by category, checking a score of each of the health-related data items depending on whether the comparison result meets a specified condition, and determining a ranking of each of the health-related data items by adding the checked scores,
select at least part of attribute information on the activity or the state based on at least part of the genetic information, wherein the attribute information comprises the priority information on the health-related data items calculated based on the genetic information and the status information,
obtain guide information associated with the health-related data items, and
provide the guide information through an output module based on at least part of the priority information, wherein the guide information is arranged based on the priority information,
wherein the category includes race, disease, physical constitution, or reproducibility with respect to the race, the disease or the physical constitution.

## Patentansprüche

1. Elektronische Vorrichtung (101, 102, 104, 201, 400, 420), umfassend:
ein Ausgabemodul;
einen Speicher (130, 405), der dazu konfiguriert ist, gesundheitsbezogene Datenelemente zu speichern, wobei jedes der gesundheitsbezogenen Datenelemente ein grafisches Objekt enthält, das einer Aktivität oder einem Zustand eines Benutzers entspricht; und
einen Prozessor (120, 210, 401, 411), der zu Folgendem konfiguriert ist:
Erlangen von Statusinformationen, die der Aktivität oder dem Zustand entsprechen, die/der mindestens einem Datenelement zugeordnet ist, das aus den gesundheitsbezogenen Datenelementen ausgewählt ist,
Erlangen von genetischen Informationen über den Benutzer,
Berechnen von Prioritätsinformationen zu den gesundheitsbezogenen Datenelementen durch Vergleichen von genetischen Informationen, die jedem der gesundheitsbezogenen Datenelemente entsprechen, mit Standardstichprobendaten nach Kategorie, Überprüfen einer Bewertung jedes der gesundheitsbezogenen Datenelemente in Abhängigkeit davon, ob das Vergleichsergebnis eine spezifische Bedingung erfüllt, und Bestimmen einer Rangfolge jedes der gesundheitsbezogenen Datenelemente durch Hinzufügen der überprüften Bewertungen,
Auswählen mindestens eines Teils von Attributinformationen zu der Aktivität oder dem Zustand basierend auf mindestens einem Teil der genetischen Informationen, wobei die Attributinformationen die Prioritätsinformationen zu den gesundheitsbezogenen Datenelementen umfassen, die basierend auf den genetischen Informationen und den Statusinformationen berechnet wurden,
Erlangen von Führungsinformationen, die den gesundheitsbezogenen Datenelementen zugeordnet sind, und
Bereitstellen der Führungsinformationen über das Ausgabemodul basierend auf mindestens einem Teil der Prioritätsinformationen, wobei die Führungsinformationen basierend auf den Prioritätsinformationen angeordnet sind,
wobei die Kategorie ethnische Zugehörigkeit, Krankheit, körperliche Konstitution oder Reproduzierbarkeit in Bezug auf die ethnische Zugehörigkeit, die Krankheit oder die körperliche Konstitution enthält.

2. Elektronische Vorrichtung nach Anspruch 1, ferner umfassend ein Kommunikationsmodul (220),
wobei der Prozessor dazu konfiguriert ist, mindestens einen Teil der genetischen Informationen von einer externen elektronischen Vorrichtung über das Kommunikationsmodul zu empfangen.

3. Elektronische Vorrichtung nach Anspruch 2, wobei der Prozessor zu Folgendem konfiguriert ist:
Empfangen verschlüsselter Daten, die mindestens einem Teil der genetischen Informationen entsprechen, als mindestens einen Teil eines Vorgangs des Empfangens des mindestens einen Teils der genetischen Informationen; und
Erlangen der genetischen Informationen durch Entschlüsseln der verschlüsselten Daten.

4. Elektronische Vorrichtung nach Anspruch 1, wobei der Prozessor dazu konfiguriert ist, die erlangten genetischen Informationen in dem Speicher zu speichern.

5. Elektronische Vorrichtung nach Anspruch 4, wobei der Speicher einen Bereich zur Sicherheitsverbesserung (SE) umfasst,
wobei der Prozessor dazu konfiguriert ist, die erlangten genetischen Informationen in dem SE-Bereich zu speichern.

6. Elektronische Vorrichtung nach Anspruch 1, ferner umfassend einen Sensor,
wobei der Prozessor dazu konfiguriert ist, mindestens einen Teil der Statusinformationen basierend auf mindestens einem Teil der über den Sensor erfassten Daten zu erlangen.

7. Elektronische Vorrichtung nach Anspruch 1, wobei der Prozessor dazu konfiguriert ist, akkumulierte numerische Informationen basierend auf mindestens einem Teil der Statusinformationen zu aktualisieren.

8. Elektronische Vorrichtung nach Anspruch 7,
wobei der Prozessor zu Folgendem konfiguriert ist:
Vergleichen der akkumulierten numerischen Informationen und der gewünschten numerischen Informationen; und
Bereitstellen einer Benachrichtigung, die der Aktivität oder dem Zustand zugeordnet ist, über das Ausgabemodul, wenn ein Vergleichsergebnis eine spezifizierte Bedingung erfüllt.

9. Computerimplementiertes Verfahren, wobei das Verfahren Folgendes umfasst:
Erlangen von Statusinformationen, die einer Aktivität oder einem Zustand eines Benutzers entsprechen, die/der mindestens einem Datenelement zugeordnet ist, das aus gesundheitsbezogenen Datenelementen ausgewählt ist;
Erlangen von genetischen Informationen über den Benutzer;
Berechnen von Prioritätsinformationen zu den gesundheitsbezogenen Datenelementen durch Vergleichen von genetischen Informationen, die jedem der gesundheitsbezogenen Datenelemente entsprechen, mit Standardstichprobendaten nach Kategorie, Überprüfen einer Bewertung jedes der gesundheitsbezogenen Datenelemente in Abhängigkeit davon, ob das Vergleichsergebnis eine spezifische Bedingung erfüllt, und Bestimmen einer Rangfolge jedes der gesundheitsbezogenen Datenelemente durch Hinzufügen der überprüften Bewertungen;
Auswählen mindestens eines Teils von Attributinformationen zu der Aktivität oder dem Zustand basierend auf den genetischen Informationen, wobei die Attributinformationen die Prioritätsinformationen zu den gesundheitsbezogenen Datenelementen umfassen, die basierend auf den genetischen Informationen und den Statusinformationen berechnet wurden;
Erlangen von Führungsinformationen, die den gesundheitsbezogenen Datenelementen zugeordnet sind; und
Bereitstellen der Führungsinformationen über ein Ausgabemodul basierend auf mindestens einem Teil der Prioritätsinformationen, wobei die Führungsinformationen basierend auf den Prioritätsinformationen angeordnet sind,
wobei die Kategorie ethnische Zugehörigkeit, Krankheit, körperliche Konstitution oder Reproduzierbarkeit in Bezug auf die ethnische Zugehörigkeit, die Krankheit oder die körperliche Konstitution enthält.

10. Verfahren nach Anspruch 9, wobei das Verfahren ferner Aktualisieren akkumulierter numerischer Informationen basierend auf mindestens einem Teil der Statusinformationen umfasst.

11. Verfahren nach Anspruch 10, wobei das Verfahren ferner Folgendes umfasst:
Vergleichen der akkumulierten numerischen Informationen und der gewünschten numerischen Informationen; und
Bereitstellen einer Benachrichtigung, die der Aktivität oder dem Zustand zugeordnet ist, über das Ausgabemodul, wenn ein Vergleichsergebnis eine spezifizierte Bedingung erfüllt.

12. Nichtflüchtiges computerlesbares Medium, das ein Computerprogramm verkörpert, wobei das Computerprogramm einen Code umfasst, der, wenn er durch mindestens einen Prozessor einer elektronischen Vorrichtung ausgeführt wird, den mindestens einen Prozessor zu Folgendem veranlasst:
Erlangen von Statusinformationen, die einer Aktivität oder einem Zustand entsprechen, die/der mindestens einem Datenelement zugeordnet ist, das aus gesundheitsbezogenen Datenelementen ausgewählt ist,
Erlangen von genetischen Informationen über den Benutzer,
Berechnen von Prioritätsinformationen zu den gesundheitsbezogenen Datenelementen durch Vergleichen von genetischen Informationen, die jedem der gesundheitsbezogenen Datenelemente entsprechen, mit Standardstichprobendaten nach Kategorie, Überprüfen einer Bewertung jedes der gesundheitsbezogenen Datenelemente in Abhängigkeit davon, ob das Vergleichsergebnis eine spezifische Bedingung erfüllt, und Bestimmen einer Rangfolge jedes der gesundheitsbezogenen Datenelemente durch Hinzufügen der überprüften Bewertungen,
Auswählen mindestens eines Teils von Attributinformationen zu der Aktivität oder dem Zustand basierend auf mindestens einem Teil der genetischen Informationen, wobei die Attributinformationen die Prioritätsinformationen zu den gesundheitsbezogenen Datenelementen umfassen, die basierend auf den genetischen Informationen und den Statusinformationen berechnet wurden,
Erlangen von Führungsinformationen, die den gesundheitsbezogenen Datenelementen zugeordnet sind, und
Bereitstellen der Führungsinformationen über ein Ausgabemodul basierend auf mindestens einem Teil der Prioritätsinformationen, wobei die Führungsinformationen basierend auf den Prioritätsinformationen angeordnet sind,
wobei die Kategorie ethnische Zugehörigkeit, Krankheit, körperliche Konstitution oder Reproduzierbarkeit in Bezug auf die ethnische Zugehörigkeit, die Krankheit oder die körperliche Konstitution enthält.

## Revendications

1. Dispositif électronique (101, 102, 104, 201, 400, 420) comprenant :
un module de sortie ;
une mémoire (130, 405) configurée pour stocker des éléments de données liés à la santé, chacun des éléments de données liés à la santé comprenant un objet graphique correspondant à une activité ou à un état d'un utilisateur ; et
un processeur (120, 210, 401, 411) configuré pour :
obtenir des informations d'état correspondant à l'activité ou à l'état associé à au moins un élément de données sélectionné parmi les éléments de données liés à la santé, obtenir des informations génétiques sur l'utilisateur, calculer des informations de priorité sur les éléments de données liés à la santé en comparant des informations génétiques correspondant à chacun des éléments de données liés à la santé avec des données d'échantillon standard par catégorie, en vérifiant un score de chacun des éléments de données liés à la santé selon que le résultat de la comparaison remplit ou non une condition spécifiée, et en déterminant un classement de chacun des éléments de données liés à la santé par addition des scores vérifiés, sélectionner au moins une partie d'informations d'attribut sur l'activité ou l'état sur la base d'au moins une partie des informations génétiques, lesdites informations d'attribut comprenant les informations de priorité sur les éléments de données liés à la santé calculées sur la base des informations génétiques et des informations d'état, obtenir des informations de guidage associées aux éléments de données liés à la santé, et
fournir les informations de guidage par l'intermédiaire du module de sortie sur la base d'au moins une partie des informations de priorité, lesdites informations de guidage étant agencées sur la base des informations de priorité, ladite catégorie comprenant la race, la maladie, la constitution physique, ou la reproductibilité par rapport à la race, à la maladie ou à la constitution physique.

2. Dispositif électronique de la revendication 1, comprenant en outre un module de communication (220), ledit processeur étant configuré pour recevoir au moins une partie des informations génétiques en provenance d'un dispositif électronique externe par l'intermédiaire du module de communication.

3. Dispositif électronique de la revendication 2, ledit processeur étant configuré pour :
recevoir des données chiffrées correspondant à au moins une partie des informations génétiques en tant qu'au moins une partie d'une opération de réception de ladite au moins une partie des informations génétiques ; et
obtenir les informations génétiques par déchiffrement des données chiffrées.

4. Dispositif électronique de la revendication 1, ledit processeur étant configuré pour stocker les informations génétiques obtenues dans la mémoire.

5. Dispositif électronique de la revendication 4, ladite mémoire comprenant une zone de renforcement de la sécurité (SE), ledit processeur étant configuré pour stocker les informations génétiques obtenues dans la zone SE.

6. Dispositif électronique de la revendication 1, comprenant en outre un capteur, ledit processeur étant configuré pour obtenir au moins une partie des informations d'état sur la base d'au moins une partie de données détectées par l'intermédiaire du capteur.

7. Dispositif électronique de la revendication 1, ledit processeur étant configuré pour mettre à jour des informations numériques accumulées sur la base d'au moins une partie des informations d'état.

8. Dispositif électronique de la revendication 7, ledit processeur étant configuré pour :
comparer les informations numériques accumulées et des informations numériques souhaitées ; et
fournir une notification associée à l'activité ou à l'état par l'intermédiaire du module de sortie lorsqu'un résultat de comparaison remplit une condition spécifiée.

9. Procédé mis en œuvre par ordinateur, le procédé comprenant :
l'obtention d'informations d'état correspondant à une activité ou à un état d'un utilisateur associé à au moins un élément de données sélectionné parmi des éléments de données liés à la santé ;
l'obtention d'informations génétiques sur l'utilisateur ;
le calcul d'informations de priorité sur les éléments de données liés à la santé en comparant des informations génétiques correspondant à chacun des éléments de données liés à la santé avec des données d'échantillon standard par catégorie, en vérifiant un score de chacun des éléments de données liés à la santé selon que le résultat de la comparaison remplit ou non une condition spécifiée, et en déterminant un classement de chacun des éléments de données liés à la santé par addition des scores vérifiés ;
la sélection d'au moins une partie d'informations d'attribut sur l'activité ou l'état sur la base des informations génétiques, lesdites informations d'attribut comprenant les informations de priorité sur les éléments de données liés à la santé calculées sur la base des informations génétiques et des informations d'état ;
l'obtention d'informations de guidage associées aux éléments de données liés à la santé ; et
la fourniture des informations de guidage par l'intermédiaire d'un module de sortie sur la base d'au moins une partie des informations de priorité, lesdites informations de guidage étant agencées sur la base des informations de priorité,
ladite catégorie comprenant la race, la maladie, la constitution physique, ou la reproductibilité par rapport à la race, à la maladie ou à la constitution physique.

10. Procédé de la revendication 9, ledit procédé comprenant en outre la mise à jour d'informations numériques accumulées sur la base d'au moins une partie des informations d'état.

11. Procédé de la revendication 10, ledit procédé comprenant en outre :
la comparaison des informations numériques accumulées et des informations numériques souhaitées ; et
la fourniture d'une notification associée à l'activité ou à l'état par l'intermédiaire du module de sortie lorsqu'un résultat de comparaison remplit ou non une condition spécifiée.

12. Support lisible par ordinateur non transitoire, incorporant un programme d'ordinateur, le programme d'ordinateur comprenant un code qui, lorsqu'il est exécuté par au moins un processeur d'un dispositif électronique, amène ledit au moins un processeur à :
obtenir des informations d'état correspondant à une activité ou à un état associé à au moins un élément de données sélectionné parmi des éléments de données liés à la santé,
obtenir des informations génétiques sur l'utilisateur, calculer des informations de priorité sur les éléments de données liés à la santé en comparant des informations génétiques correspondant à chacun des éléments de données liés à la santé avec des données d'échantillon standard par catégorie, en vérifiant un score de chacun des éléments de données liés à la santé selon que le résultat de la comparaison remplit ou non une condition spécifiée, et en déterminant un classement de chacun des éléments de données liés à la santé par addition des scores vérifiés, sélectionner au moins une partie d'informations d'attribut sur l'activité ou l'état sur la base d'au moins une partie des informations génétiques, lesdites informations d'attribut comprenant les informations de priorité sur les éléments de données liés à la santé calculées sur la base des informations génétiques et des informations d'état, obtenir des informations de guidage associées aux éléments de données liés à la santé, et
fournir les informations de guidage par l'intermédiaire d'un module de sortie sur la base d'au moins une partie des informations de priorité, lesdites informations de guidage étant agencées sur la base des informations de priorité,
ladite catégorie comprenant la race, la maladie, la constitution physique, ou la reproductibilité par rapport à la race, à la maladie ou à la constitution physique.
